# EUROPEAN PATENT SPECIFICATION

(11) **EP 4 380 673 B1**
(45) Date of publication and mention of the grant of the patent: **02.09.2026**
(21) Application number: 22854044.9
(22) Date of filing: 01.08.2022
(51) Int. Cl.: A61N 1/04, A61B 5/11, A61N 1/36

(54) **WEARABLE NEUROSTIMULATION SYSTEM**
TRAGBARES NEUROSTIMULATIONSSYSTEM
SYSTÈME DE NEUROSTIMULATION PORTABLE

(30) Priority: 03.08.2021 US 202163203895 P; 08.10.2021 US 202163262331 P; 23.11.2021 US 202163264498 P; 01.07.2022 US 202263367577 P
(43) Date of publication of application: 12.06.2024
(60) Divisional application: 26190384.3
(73) Proprietor: Cala Health, Inc., San Mateo, CA 94404 (US)
(72) Inventor: SCHULTE, Gregory T., San Mateo, California 94404 (US); BOUCH, Dustin, San Mateo, California 94404 (US); WELLS, Kyra, San Mateo, California 94404 (US); LE, Harrison, San Mateo, California 94404 (US); BALBAKY, Sami, San Mateo, California 94404 (US); HARSHBARGER, Deanna, San Mateo, California 94404 (US); PRINCE, Toni-Moi, San Mateo, California 94404 (US); FRANCISCO, Tony, San Mateo, California 94404 (US); KENT, Alexander R., San Mateo, California 94404 (US); LI, Shengzhi, San Mateo, California 94404 (US); LIBERATORE, Jessica M., San Mateo, California 94404 (US); HAMNER, Samuel Richard, San Mateo, California 94404 (US); SHUGHART, Mark, San Mateo, California 94404 (US); OZTURK, Musa, San Mateo, California 94404 (US); ROSENBLUTH, Kathryn H., San Mateo, California 94404 (US)
(74) Representative: J A Kemp LLP
(86) International application number: PCT/US2022/074376
(87) International publication number: WO 2023/015158

(56) References cited:
- US-A1- 2017 165 485
- US-A1- 2021 244 940
- US-A1- 2022 016 413
- US-B2- 10 765 856
- US-B2- 9 731 126
- US-B2- 9 802 041

## Description

### TECHNICAL FIELD

Some embodiments of the invention relate generally to systems, devices, methods for neuromodulating (such as stimulating) nerves, and methods of manufacture, and more specifically relate to wearable devices that include a band for releasably securing a stimulator on a user's limb or other body part for electrically stimulating peripheral nerve(s) to treat various diseases and disorders.

### BACKGROUND

A wide variety of modalities can be utilized to neuromodulate peripheral nerves. For example, Applicant's own work has demonstrated that electrical energy can be delivered transcutaneously via electrodes on the skin surface with neurostimulation systems to stimulate peripheral nerves, such as the median, radial, and/or ulnar nerves in the upper extremities; the tibial, saphenous, and/or peroneal nerve in the lower extremities; or the auricular vagus, auriculotemporal, trigeminal or cranial nerves on the head or ear, as non-limiting examples. A number of conditions, such as tremors, can be treated through some form of transcutaneous, percutaneous, or other implanted forms of peripheral nerve stimulation. Document US-B-10 765 856 discloses a wearable neurostimulation system.

### SUMMARY

Wearable systems to neuromodulate nerves with compact, ergonomic form factors are needed to enhance efficacy, compliance, and/or comfort with using the devices. The devices can be attached to a band that is wrapped around a patient's wrist. The band is worn throughout the day including during daily activities. To provide the ability for the patient to change or replace the band while keeping the same device, the device is not permanently coupled to the band. This feature allows the patient to swap out or upgrade their band without incurring the cost of purchasing a new device. This feature further allows the patient to switch their device between bands that are designed for different purposes and/or activities. Characteristics of each band (e.g., material, weight, size, color, etc.) can be optimized or selected depending on the purpose or activity. The patient may simply disengage their device from their current band and then engage the same device on another band that has been optimized for the upcoming activity. The presently-claimed invention is defined in claim 1. The methods described in this document are not part of the presently-claimed invention.

In some embodiments, because the device and band are used during daily activities and are not permanently coupled together, the engagement between the band and the device needs to be secure while also being ergonomic in allowing the patient to easily remove the device from the band without relying on hand tools (e.g., screwdriver, wrench, pliers). Simple attachment structures such as snaps may not provide the desired level of securement between the device and the band.

Some embodiments of the system disclosed herein include a band made from one or more soft materials (e.g., silicone, fabric). In contrast, the device can be a firm or rigid material for housing electronics. In this way, the soft goods (e.g., the band) are separate from the electronics of the device. Another advantage of the soft goods being separable from the device allows the user to personalize the size, feel, and/or aesthetics of the of the band without having to replace the device.

Another advantage of some of the systems disclosed herein is to employ an over-molded silicone band. In some embodiments, the band is formed by molding silicone over the electrodes. The over-molding process can tightly control surface variations between the band and the electrodes. For example, the degree which the electrode protrudes or is recessed relative to a strap portion of the band can be optimized. This optimization can result in improved patient comfort, increased band durability, and/or increased protection from contact with liquid.

Another advantage of some of the systems disclosed herein is to allow the user to easily interchange the device and the band. The user can remove the more costly device from the less costly band. For example, in some embodiments, the user only needs to apply a nominal force to the top surface of the device to pop the device out of the band. Separating the device from the band is also easier because of the flexibility of the band. The shape of the band adjusts and conforms to the shape of the device which prevents tight pinch points from forming when the device is engaged with the band. Without tight pinch points, the level of force required from the user to separate the band from the device is consistent over time.

Because of the interchangeable feature, the user can swap out bands depending on the expected situation in which the user expects to use the band and/or the desired comfort level when wearing the band. The interchangeable feature allows the user to also swap out bands depending on a desired electrode configuration. For example, the user can select a band that has a desirable number of electrodes and/or location of electrodes on the band. Different arrangements of electrodes on the bands can target different nerves and/or anatomical structures of the user. For example, some bands can have electrodes which not only target different nerves but also target specific characteristics of the nerves. These characteristics can include, for example, variations in nerve size, depth, and or location on the body. In some embodiments, the device comprises, consists or consists essentially of 3, 6, 9 or 12 electrodes or between 1-2 electrodes.

Another advantage of some of the systems disclosed herein is the device can select a subset of multiple electrodes for the stimulation session. The subset of electrodes can be selected depending on the desired characteristics of a specific treatment session and/or power efficiency of the electrodes. For example, the subset of electrodes can be selected and or changed depending on a real-time battery level of the device. In some embodiments, machine learning is employed.

Another advantage is the system preserves battery life. For example, in some embodiments, the electrodes that complete the electrical circuit are located in close proximity to each other (e.g., reducing electrical resistance through the user) which allows a low current (e.g., 2 mA) to effectively neuromodulate the target nerve(s) and/or anatomical structures of the user while preserving battery life.

In some embodiments, magnets are employed solely or combination with other engagement structures to attach or lock the device to the band as well as can be employed in combination with other structures (e.g., hooks, tangs, lips, slots, keyways, etc.) for securing the device to the band. The magnets can be employed in combination with snap-fit engagement between the band and the device.

Another advantage is the system is efficient and can be provided at a lower cost. For example, the band can be provided as a disposable device for use during a trial period by the user. Once the trial period is over, the user can dispose of the band. The more costly device can be repurposed for another user during their trial period.

In some embodiments, any of the devices or methods are used for treatment of depression (including but not limited to post-partum depression, depression affiliated with neurological diseases, major depression, seasonal affective disorder, depressive disorders, etc.), inflammation (e.g., neuroinflammation), Lyme disease, stroke, neurological diseases (such as Parkinson's and Alzheimer's), and gastrointestinal issues (including those in Parkinson's disease).

In several embodiments, one or more of bradykinesia, dyskinesia, gait dysfunction, dystonia and/or rigidity are treated with the devices and methods described herein (e.g., in connection with Parkinson's disease or in connection with other disorders). Rehabilitation of movement is treated in some embodiments (for example to restore or improve movement and motion) in subjects who have suffered from an acute or chronic event including, for example, cardiac events (such as atrial fibrillation, hypertension, and stroke), inflammation, neuroinflammation, etc. Epilepsy is treated in one embodiment. Treatment of movement disorders herein also includes, for example, treatment of involuntary and/or repetitive movements, such as tics, twitches, etc. (including, but not limited to, Tourette Syndrome, tic disorders for example). Rhythmic and/or non-rhythmic involuntary movements may be controlled in several embodiments. Involuntary vocal tics and other vocalizations may also be treated. Rehabilitation of movement can include, for example, rehabilitation of limb movement. In some embodiments, provided herein are treatments of restless leg syndrome, periodic limb movement disorder, repetitive movements of the limbs and abnormal sensation. Devices described herein can be placed, for example, on the wrist or leg (or both) to treat leg disorders. One or more nerves may be treated including for example, peroneal, saphenous, tibial, femoral, and sural. In some embodiments, two, three or more nerves are treated. In some embodiments, the median nerve is modulated (e.g., stimulated) along with one, two or more other nerves. A band or other device may be placed on a wrist and the leg, only on the wrist or leg, or on two or more locations on one or both limbs. A single device, two or more devices that are coupled physically and/or in communication with each other may be used. Stimulation may be automated, user-controllable, or both.

In some embodiments, disorders and symptoms caused or exacerbated by microbial infections (e.g., bacteria, viruses, fungi, and parasites) are treated. Symptoms include but are not limited to sympathetic/parasympathetic imbalance, autonomic dysfunction, inflammation (e.g., neuroinflammation), inflammation, motor and balance dysfunction, pain and other neurological symptoms. Disorders include but are not limited to tetanus, meningitis, Lyme disease, urinary tract infection, mononucleosis, chronic fatigue syndrome, autoimmune disorders, etc. In some embodiments, autoimmune disorders and/or pain unrelated to microbial infection is treated, including for example, inflammation (e.g., neuroinflammation), headache, back pain, joint pain and stiffness, muscle pain and tension, etc.

A wearable system for modulating one or more peripheral nerves of a user is provided. The system comprises a band having a frame with an engagement structure. The band has an outside and an inside, the outside being viewable by the user and the inside facing skin of the user when the band is worn by the user. The system further comprises a neurostimulation device having an upper surface, a lower surface, and an outer wall disposed therebetween. The outer wall is sized and shaped to be secured against the engagement structure when the neurostimulation device is inserted into the frame from the inside of the band while preventing the neurostimulation device from passing entirely through the frame.

In some embodiments, the engagement structure is an abutment surface, and wherein the neurostimulation device comprises a contact surface shaped and sized to contact the abutment surface when the neurostimulation device is secured to the band.

In some embodiments, the engagement structure is an opening. In some embodiments, at least a portion of the outer wall is curved between the upper and lower surfaces. In some embodiments, at least a portion of the outer wall is flat between the upper and lower surfaces. In some embodiments, the outer wall has a shape of a step, the step comprising a riser and tread, and wherein a circumference of the riser is less than an inner circumference of the opening. In some embodiments, only a portion of the outer wall has a circumference that is greater than an inner circumference of the opening.

In some embodiments, the portion of the outer wall has a conical shape. In some embodiments, the portion of the outer wall has a step shape. In some embodiments, the band includes a mechanical coupling and an electrical coupling with the frame. In some embodiments, the band comprises a first strap portion coupled to a second strap portion, the first strap portion being manufactured from silicone and the second strap portion being manufactured from fabric. In some embodiments, the band is flexible.

In some embodiments, the band further comprises an electrode system having an inner side and an outer side, the inner side comprising at least one electrode for each nerve to be modulated. In some embodiments, the outer side comprises one or more electrodes. In some embodiments, one or more electrodes disposed on the outer side are configured as sensors for measuring physiological data. For example, an electrode disposed on the outer side can be used as a sensor to contact a selected location on the user's body. In some embodiments, the band is configured to be tightened about a limb forcing the at least one electrode firmly against the skin of the user. In some embodiments, the electrode system comprises one or more electrical traces extending between the frame and the at least one electrode.

A wearable system for modulating one or more peripheral nerves of a user is provided. The system comprises a band having an outside and an inside. The outside is viewable by the user when the band is worn by the user. The inside faces skin of the user when the band is worn by the user. The system further comprises a frame coupled to the band and can have an opening and a neurostimulation device having a screen on an upper surface. The neurostimulation device can be sized and shaped so only a portion of the neurostimulation device fits within the opening when the neurostimulation device is inserted into the frame from the inside of the band. The screen can be viewable within the opening from the outside of the band.

In some embodiments, the band further comprises an electrode system having an inner side and an outer side, the inner side comprising at least one electrode for each nerve to be modulated. In some embodiments, the at least one electrode comprises at least a first electrode and a second electrode, the first electrode being configured to stimulate the median nerve of the user and the second electrode being configured to stimulate the radial or ulnar nerve of the user. In some embodiments, the at least one electrode comprises a return or ground electrode configured to be electrically coupled to the user. In some embodiments, the band comprises a first strap portion coupled to a second strap portion, the first strap portion being manufactured from silicone and the second strap portion being manufactured from fabric.

In some embodiments, a wearable system for modulating one or more peripheral nerves of a user is provided. The system can, for example, comprise, consist, or consist essentially of a neurostimulation device having a lower surface, an upper surface, and a screen. The screen can be disposed on the upper surface. At least a portion of the lower surface can contact a limb of the user when the system is worn by the user. The system can further comprise a band configured to capture the neurostimulation device against the limb so that the portion of the lower surface is in contact with the limb and the screen is viewable by the user.

In some embodiments, at least a portion of the neurostimulation device is disposed between a surface of the band and the limb, the surface contacting the neurostimulation device. In some embodiments, at least a portion of the neurostimulation device forms a press-fit with the band. In some embodiments, the band comprises a frame sized and shaped to engage the neurostimulation device. In some embodiments, the neurostimulation device comprises an outer wall configured to engage the frame. In some embodiments, the outer wall forms a step in a direction from the lower surface to the upper surface, the step comprising a riser and a tread, the tread being disposed between the frame and the limb to prevent the neurostimulation device from passing entirely through the frame when the neurostimulation is being captured by the band. In some embodiments, the outer wall has a tapering conical shape in a direction from the lower surface to the upper surface, the tapering conical shape preventing the neurostimulation device from passing entirely through the frame when the neurostimulation is being captured by the band. In some embodiments, the band further comprises an electrode system having an inner side and an outer side, the inner side comprising at least one electrode for each nerve to be modulated.

A wearable system for modulating one or more peripheral nerves of a user is provided. The system comprises of a band having an outside and an inside, the outside being viewable by the user when the band is worn by the user, the inside facing skin of the user when the band is worn by the user. The system comprises a frame coupled to the band and can have an abutment surface and an opening and a neurostimulation device having a contact surface. The neurostimulation device can be insertable from the inside of the band into the opening so that the contact surface abuts the abutment surface of the frame preventing the neurostimulation device from passing entirely through the opening and exiting the opening on the outside of the band.

In some embodiments, a band for releasably securing a neurostimulation device to a limb of a user is provided. The neurostimulation device can be configured to generate a signal for modulating one or more peripheral nerves of the user. The band can, for example, comprise, consist, or consist essentially of a strap having an outside and an inside, the outside being viewable by the user and the inside facing skin of the user when the band is secured to the limb and a frame coupled to the strap and having an opening. The opening can be sized and shaped relative to the neurostimulation device to secure the neurostimulation device relative to the frame while preventing the entire neurostimulation device from passing through the opening when the neurostimulation device is inserted into the opening from the inside of the strap.

In some embodiments, a method of releasably securing a neurostimulation device to a band is provided. The band can have a frame with an opening. The opening can be sized and shaped relative to the neurostimulation device to secure the neurostimulation device relative to the frame while preventing the entire neurostimulation device from passing through the opening. The neurostimulation device can be configured to generate a signal for modulating one or more peripheral nerves of a user. The method can comprise inserting the neurostimulation device into the opening in a direction to secure the neurostimulation device and removing the neurostimulation device from the opening in the direction.

In some embodiments, a method of releasably securing a neurostimulation device to a band is provided. The band can, for example, comprise, consist, or consist essentially of an outside and an inside with the outside being viewable by a user and the inside facing skin of the user when the band is secured to a limb of the user. The method can comprise inserting at least a portion of the neurostimulation device into an opening in a frame of the band from the inside of the band and abutting a contact surface of the neurostimulation device against an abutment surface of the frame so that the neurostimulation device is secured by the band while preventing the entire neurostimulation device from continuing through the opening and exiting the opening on the outside of the band.

In some embodiments, a band for releasably securing a neurostimulation device to a limb of a user is provided. The neurostimulation device can be configured to generate a signal for modulating one or more peripheral nerves of the user and comprise means for inserting at least a portion of the neurostimulation device into an opening in a frame of the band from the inside of the band and means for abutting the neurostimulation device against the frame so that the neurostimulation device is secured by the band while preventing the entire neurostimulation device from continuing through the opening and exiting the opening on the outside of the band.

In some embodiments, a band configured to secure a neurostimulation system on a wrist of a user is provided. The band can, for example, comprise, consist, or consist essentially of an outer surface and an inner surface with the inner surface being configured to be in contact with the wrist of the user. The band can comprise a first portion adjacent to a first end along a length of the band. The first portion can be configured to releasably engage the neurostimulation system. The first portion can comprise a connection and an aperture on opposite sides of the first portion. A second portion along the length of the band can comprise an electrode system having at least one electrode on the inner surface of the band for each nerve to be stimulated. A third portion along the length of the band can be configured to pass through the aperture in the first portion and fold back on itself. A fourth portion adjacent to the third portion along the length of the band can comprise an attachment mechanism for securing the fourth portion to an outer surface of the band.

In some embodiments, a wrist wearable system configured to removably secure a controller is provided. The system can, for example, comprise, consist, or consist essentially of an inside and an outside with the inside being configured to be in contact with a wrist of a user. The wrist wearable system can comprise a frame comprising an engagement structure configured to receive the controller from the inside of the system to engage and secure the controller and a strap extending from a first portion of the frame, wherein an end of the strap is not secured to the frame.

In some embodiments, a wearable system for transcutaneously delivering electrical signals to one or more nerves of a user is provided. The system can have a durable component and a replaceable component comprising at least one electrode. The replaceable component can be configured to maintain the durable component and the at least one electrode in contact with skin of the user by applying a force to the durable component in a direction towards the skin.

In some embodiments, the at least one electrode contacts the skin of the user at a location different than where the durable component contacts the skin of the patient. In some embodiments, the at least one electrode comprises a first electrode and a second electrode, the first electrode being configured to stimulate a median nerve of the user and the second electrode being configured to stimulate a radial or ulnar nerve of the user. In some embodiments, the at least one electrode comprises a return or ground electrode configured to be electrically coupled to the user. In some embodiments, the durable component is able to withstand more use than the replaceable component. In some embodiments, the durable component has a useful life greater than a useful life of the replaceable component. In some embodiments, the direction is perpendicular to the skin of the user.

In some embodiments, the replaceable component comprises a band configured to encircle a limb of the user. In some embodiments, the replaceable component comprises a frame, the frame contacting the durable component when the replaceable component maintains the durable component in contact with the skin of the user. In some embodiments, the frame comprises a receptacle, the receptacle being sized and shaped to receive at least a portion of the durable component. In some embodiments, the durable component comprises a screen. In some embodiments, the screen is visible to the user when the durable component is in contact with the skin of the user.

In some embodiments, the system further comprises an electrical coupling between the replaceable component and the durable component, the electrical coupling being inaccessible when the replaceable component is maintaining contact between the durable component and the skin of the user. In some embodiments, the electrical coupling comprises an electrical interconnect. In some embodiments, the electrical interconnect is spring-loaded. In some embodiments, the electrical interconnect moves from a retracted position to an extended position when the durable component is removed from the replaceable component.

In some embodiments, the system comprises a mechanical coupling between the replaceable component and the durable component. In some embodiments, the mechanical coupling comprises an engagement structure. In some embodiments, the mechanical coupling comprises an abutment surface. In some embodiments, the mechanical coupling comprises a contact surface. In some embodiments, the mechanical coupling comprises an opening. In some embodiments, the mechanical coupling is configured to inhibit removal of the durable component from the disposable component in the absence of the force.

In some embodiments, a magnitude of a force applied by the user to remove the durable component from the disposable component is less than a magnitude of the force applied by the disposable component to maintain the durable component in contact with the skin of the user. In some embodiments, a direction of the force which removes the durable component from the disposable component is parallel to a direction of the force which maintains the durable component in contact with the skin of the user. In some embodiments, the disposable component comprises a first strap portion coupled to a second strap portion, the first strap portion being manufactured from silicone and the second strap portion being manufactured from fabric.

In some embodiments, the disposable component is flexible. In some embodiments, the disposable component comprises an electrode system having an inner side and an outer side, the inner side comprising the at least one electrode. In some embodiments, the disposable component is configured to be tightened about a limb of the user. In some embodiments, the tightened disposable component forces the at least one electrode firmly against the skin of the user.

In some embodiments, the electrode system comprises one or more electrical traces. In some embodiments, the one or more electrical traces are in electrical contact with the at least one electrode. In some embodiments, the one or more electrical traces are in electrical contact with the durable component at least when the replaceable component is maintaining the durable component in contact with the skin of the user. In some embodiments, at least a portion of the durable component forms a press-fit with the disposable component. In some embodiments, the durable component is a neurostimulation device. In some embodiments, the electrical signals delivered to the one or more nerves of the user block nerve signals. In some embodiments, the electrical signals delivered to the one or more nerves of the user stimulate nerve signals.

In some embodiments, a wearable system for transcutaneously delivering electrical signals to one or more nerves of a user is provided. The system has a first component comprising at least one electrical interconnect. The system has a second component comprising at least one electrical interconnect positioned so as to contact the at least one electrical interconnect of the first component when the second component is selectively engaged with the first component. The second component can be configured to maintain the first component in contact with skin of the user when worn by the user.

In some embodiments, the second component comprises at least one electrode. In some embodiments, the at least one electrode contacts the skin of the user at a location different than where the first component contacts the skin of the patient. In some embodiments, the at least one electrode comprises a first electrode and a second electrode, the first electrode being configured to stimulate a median nerve of the user and the second electrode being configured to stimulate a radial or ulnar nerve of the user. In some embodiments, the at least one electrode comprises a return or ground electrode configured to be electrically coupled to the user. In some embodiments, the first component is able to withstand more use than the second component. In some embodiments, the first component has a useful life greater than a useful life of the replaceable component. In some embodiments, the direction is perpendicular to the skin of the user. In some embodiments, the second component comprises a band configured to encircle a limb of the user.

In some embodiments, the second component comprises a frame, the frame contacting the first component when the second component maintains the first component in contact with the skin of the user. In some embodiments, the frame comprises a receptacle, the receptacle being sized and shaped to receive at least a portion of the first component. In some embodiments, the first component comprises a screen. In some embodiments, the screen is visible to the user when the first component is in contact with the skin of the user. In some embodiments, the at least one electrical interconnect is spring-loaded. In some embodiments, the at least one electrical interconnect moves from a retracted position to an extended position when the first component is removed from the second component.

In some embodiments, the system comprises a mechanical coupling between the second component and the first component. In some embodiments, the mechanical coupling comprises an engagement structure. In some embodiments, the mechanical coupling comprises an abutment surface. In some embodiments, the mechanical coupling comprises a contact surface. In some embodiments, the mechanical coupling comprises an opening. In some embodiments, the second component is configured to apply a force to the first component in a direction towards the skin when worn by the user. In some embodiments, the mechanical coupling is configured to inhibit removal of the first component from the second component in the absence of the force. In some embodiments, a magnitude of a force applied by the user to remove the first component from the second component is less than a magnitude of the force applied by the second component to maintain the first component in contact with the skin of the user.

In some embodiments, a direction of the force which removes the first component from the second component is parallel to a direction of the force which maintains the first component in contact with the skin of the user. In some embodiments, the second component comprises a first strap portion coupled to a second strap portion, the first strap portion being manufactured from silicone and the second strap portion being manufactured from fabric. In some embodiments, the second component is flexible. In some embodiments, the second component comprises an electrode system having an inner side and an outer side, the inner side comprising the at least one electrode. In some embodiments, the second component is configured to be tightened about a limb of the user. In some embodiments, the tightened second component forces the at least one electrode firmly against the skin of the user.

In some embodiments, the electrode system comprises one or more electrical traces. In some embodiments, the one or more electrical traces are in electrical contact with the at least one electrode. In some embodiments, the one or more electrical traces are in electrical contact with the first component at least when the second component is maintaining the first component in contact with the skin of the user. In some embodiments, the at least one electrical interconnect of the first component and the at least one electrical interconnect of the second component are inaccessible when the second component is selectively engaged with the first component.

In some embodiments, at least a portion of the first component forms a press-fit with the second component. In some embodiments, the first component is a neurostimulation device. In some embodiments, the electrical signals delivered to the one or more nerves of the user block nerve signals. In some embodiments, the electrical signals delivered to the one or more nerves of the user stimulate nerve signals.

In some embodiments, the electrical signals delivered to the one or more nerves of the user varies a burst frequency after a prespecified time period. In some embodiments, the electrical signals delivered to the one or more nerves of the user varies a burst frequency after a prespecified number of bursts. In some embodiments, the electrical signals delivered to the one or more nerves of the user varies a pulse frequency after a prespecified time period. In some embodiments, the electrical signals delivered to the one or more nerves of the user varies a pulse frequency after a prespecified number of bursts.

In some embodiments, a system for providing therapy recommendations to a user is provided. In some embodiments, the system comprises one or more hardware processors configured to: receive kinematic data and/or patient satisfaction ratings of an assessment period; display a tremor improvement score based at least in part on the kinematic data and/or the patient satisfaction ratings of the assessment period; and/or provide a plurality of waveform patterns for selection by the user.

In some embodiments, a method for providing therapy recommendations to a user is provided. In some embodiments, the method comprises receiving kinematic data and/or patient satisfaction ratings of an assessment period; displaying a tremor improvement score based at least in part on the kinematic data and/or the patient satisfaction ratings of the assessment period; and/or providing a plurality of waveform patterns for selection by the user.

### BRIEF DESCRIPTION OF THE DRAWINGS

The following drawings are for illustrative purposes only and show non-limiting embodiments. Features from different figures may be combined in several embodiments.
**Figure 1** is a picture of a system that includes a device and band that is worn by a user. The device is detachably coupled to the band for providing transcutaneous peripheral nerve stimulation to the user.
**Figure 2** is another picture of the system from Figure 1 taken from a side of the system.
**Figure 3** is a perspective view of the system from Figure 1 showing an electrode system on an inside of the band.
**Figure 4** is a perspective side view of the system with the device aligned with an opening in the frame prior to inserting the device into the opening to secure the device to the band.
**Figure 5** is a perspective view of the device from Figure 4.
**Figure 6** is a perspective view of the band from Figure 4.
**Figures 7-10** are views similar to Figures 3-6, respectively.
**Figures 11-14** are views similar to Figures 3-6, respectively.
**Figures 15-18** are views similar to Figures 3-6, respectively.
**Figure 19** is a perspective view of a system similar to the system from Figure 1 showing an electrode system on an inside of the band.
**Figure 20** is a top plan view of the system from Figure 19.
**Figure 21** is a side view of the system from Figure 19.
**Figure 22** is a bottom perspective of the system from Figure 19.
**Figure 23** is a perspective top, side view of the system with the device aligned with an opening in the frame prior to inserting the device into the opening to secure the device to the band.
**Figure 23A** is a partial view of the frame from Figure 23 showing one or more electrical contacts on the frame.
**Figure 24** is a bottom perspective view of the system with the band removed.
**Figure 25** includes views of the system in Figure 24.
**Figure 26** is another bottom perspective view of the system with the band removed.
**Figure 27** includes views of the system in Figure 26.
**Figure 28** is a front-right perspective view of a system similar to Figures 1-6.
**Figure 29** is a rear-left perspective view of the system of Figure 28.
**Figure 30** is a rear elevational view of the system of Figure 28.
**Figure 31** is a front elevational view of the system of Figure 28.
**Figure 32** is a right side elevational view of the system of Figure 28.
**Figure 33** is a left side elevational view of the system of Figure 28.
**Figure 34** is a top plan view of the system of Figure 28.
**Figure 35** is a bottom plan view of the system of Figure 28.
**Figure 36** is a front-right perspective view of a device from Figure 28.
**Figure 37** is a rear-left perspective view of the device from Figure 28.
**Figure 38** is a rear elevational view of the device from Figure 28.
**Figure 39** is a front elevational view of the device from Figure 28.
**Figure 40** is a right side elevational view of the device from Figure 28.
**Figure 41** is a left side elevational view of the device from Figure 28.
**Figure 42** is a top plan view of the device from Figure 28.
**Figure 43** is a bottom plan view of the device from Figure 28.
**Figure 44** is a front-right perspective view of a band from Figure 28.
**Figure 45** is a rear-left perspective view of the band from Figure 28.
**Figure 46** is a rear elevational view of the band from Figure 28.
**Figure 47** is a front elevational view of the band from Figure 28.
**Figure 48** is a right side elevational view of the band from Figure 28.
**Figure 49** is a left side elevational view of the band from Figure 28.
**Figure 50** is a top plan view of the band from Figure 28.
**Figure 51** is a bottom plan view of the band from Figure 28.
**Figure 52A** illustrates an example of a block diagram of the neuromodulation (e.g., neurostimulation) devices disclosed herein.
**Figure 52B** illustrates a block diagram of an embodiment of a user interface device that can be implemented with the hardware components described herein.
**Figure 53** illustrates a block diagram of an embodiment of a device and system that provides peripheral nerve stimulation and senses a biological or kinematic measure and/or receives user satisfaction data that is used to customize or modify the delivery of an electrical stimulus.
**Figures 54A****-C2** illustrate examples of how stimulation parameters (e.g., burst frequency, pulse frequency, and pulse phase) are varied between two or more prespecified values as stimulation is alternated across two nerves (e.g., median and radial or ulnar nerves).
**Figures 54D1-E** illustrate examples of how stimulation parameters (e.g., pulse frequency) are varied between two or more values based on physiological parameters (e.g., tremor frequency and respiration rate) as stimulation is alternated across two nerves (e.g., median and radial or ulnar nerves).

### DETAILED DESCRIPTION

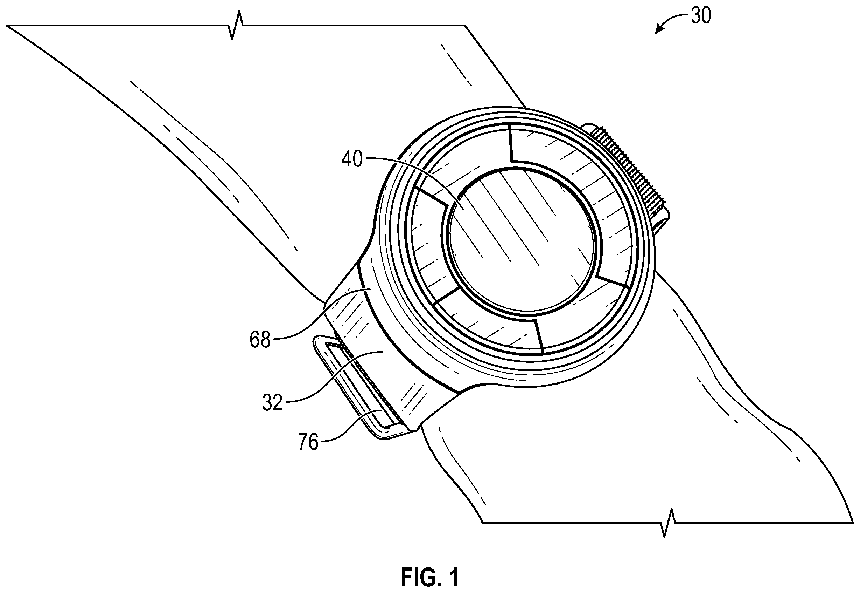

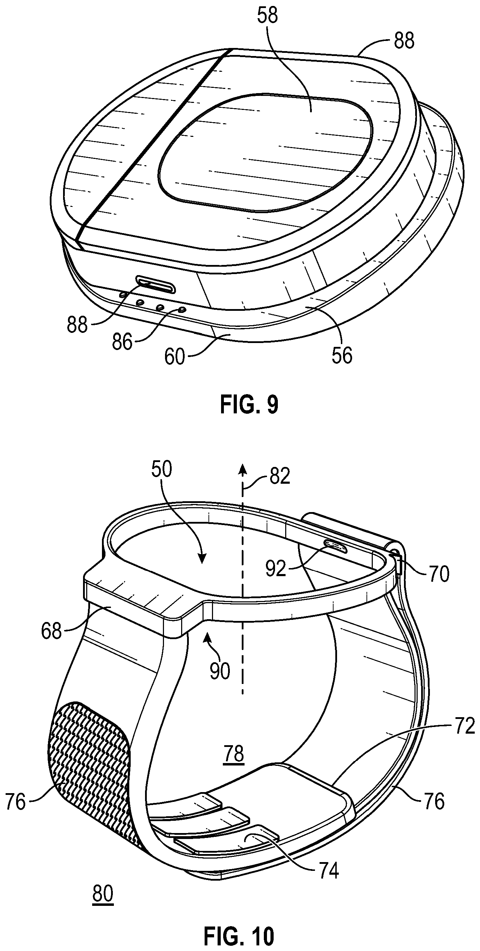

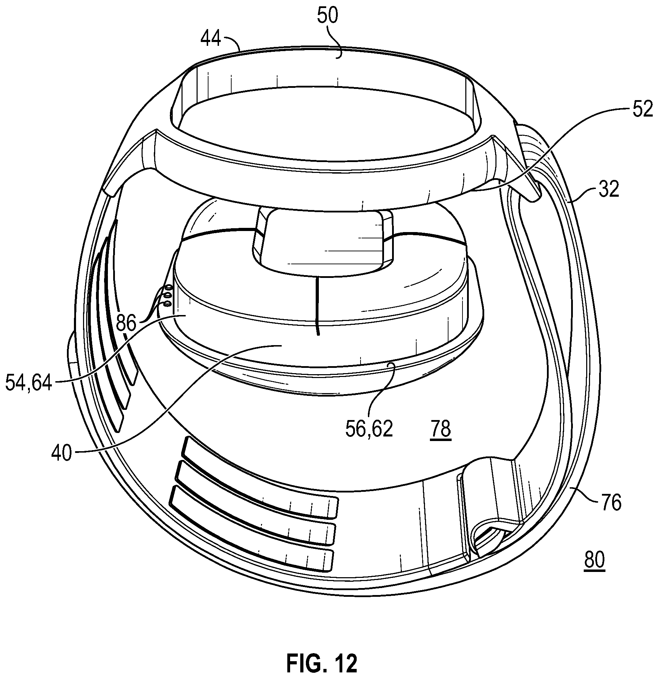

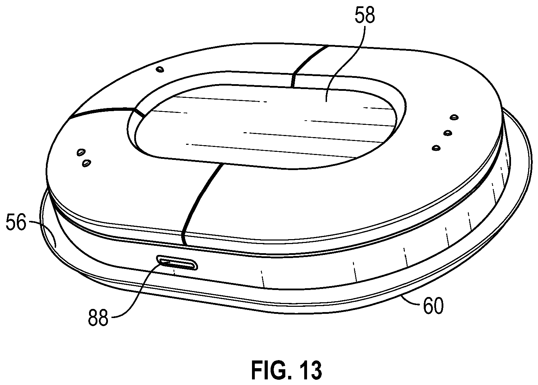

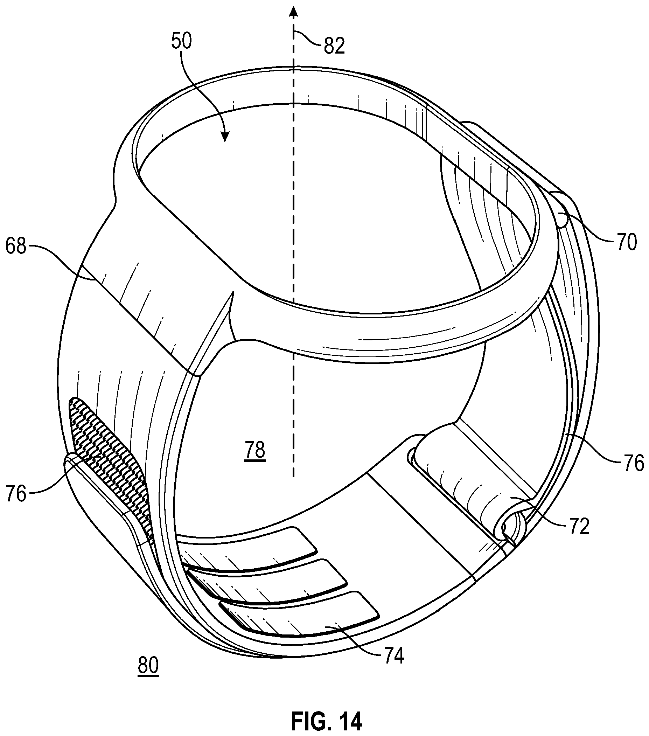

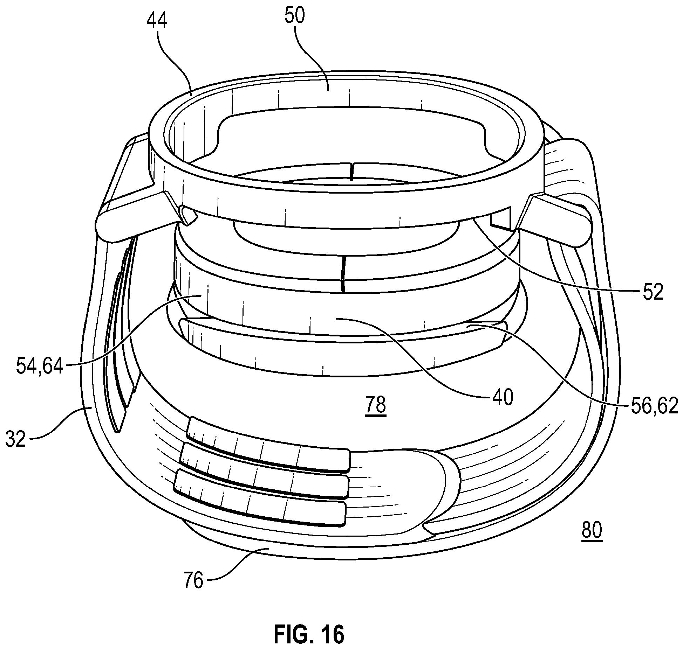

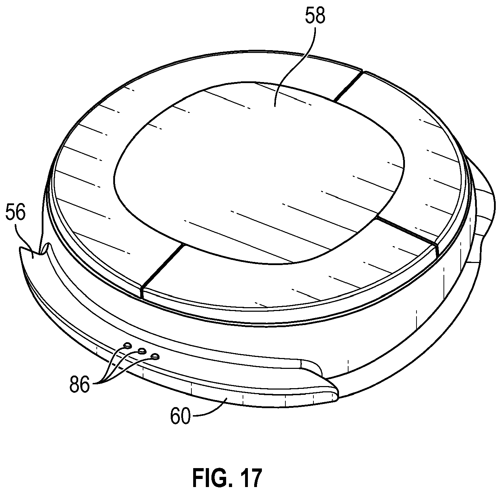

Disclosed herein is a system that includes band for securing a device configured for providing neuromodulation (e.g., neurostimulation). The device may be configured to be coupled to the surface of a user's skin for transcutaneous stimulation using the band. The system can comprise any combination of features disclosed in any of the figures. Accordingly, the system can have any number of different configurations. Thus, while certain combinations of features are illustrated in each figure, the features are not limited to only being incorporated as part of the illustrated combinations. In this way, any of the features disclosed in any of the figures can be employed with any other feature disclosed in any of the figures. For simplicity of description, certain combinations of features were selected to be illustrated in any given figure. However, the selected combinations of features do not limit the disclosure. Accordingly, any of the features illustrated in **Figures 1 to 54E** can be combined in any way.

The bands provided herein may be configured to secure the device to the user. The devices provided herein may be configured to stimulate peripheral nerves of the user when secured by the band. The neuromodulation (e.g., neurostimulation) devices may be configured to transmit one or more neuromodulation (e.g., neurostimulation) signals across the skin of the user. In many embodiments, the devices are wearable devices configured to be worn by a user. The user may be a human, another mammal, or other animal user. The system could also include signal processing systems and methods for enhancing diagnostic and therapeutic protocols relating to the same.

In some embodiments, the device is configured to be wearable on an upper extremity of a user (e.g., a wrist, forearm, arm, and/or finger(s) of a user). In some embodiments, the device is configured to be wearable on a lower extremity (e.g., ankle, calf, knee, thigh, foot, and/or toes) of a user. In some embodiments, the device is configured to be wearable on the head or neck (e.g., forehead, ear, neck, nose, and/or tongue). Single or multiple bands that partially or fully encircle a limb (such as a wrist, ankle, arm, leg) are provided in some embodiments. Ear devices are also provided in some embodiments that can be used with or without a limb band. In one embodiment, an ear device and a wrist band are provided for synergistic treatment.

In some embodiments, the device is configured to be wearable on or proximate an ear of a user, including but not limited to auricular neuromodulation (e.g., neurostimulation) of the auricular branch of the vagus nerve, for example. In some embodiments, the vagus nerve, trigeminal nerve and/or great auricular nerve is/are neuromodulated. In some embodiments, only the vagus nerve is neuromodulated. In some embodiments, the vagus nerve and one, two or more other nerves are neuromodulated (e.g., trigeminal nerve, greater auricular nerve, nerves of the auricular branch, auricular branch of the vagus nerve, the facial nerve, the auriculotemporal nerve etc.). In some embodiments, the vagus nerve is not stimulated and instead, for example, another nerve is stimulated (e.g., trigeminal nerve, great auricular nerve, the facial nerve, the auriculotemporal nerve, other nerves of the auricular branch, etc.). An auricular (e.g., ear) device can include an earpiece or bud for one or more portions of the ear such as an ear canal or external ear. In some embodiments, the device can include a housing or enclosure (e.g., miniaturized) that is attached to a portion of the user (for example, secured behind the ear, wrapped around the ear, secured in the ear, secured over the ear, in a headband secured around the user's head, around a neck of the user, and/or around an arm of the user). One to six or more electrodes may be placed on the earpiece or bud, or on a device connected to the earpiece/bud. In some embodiments, only a portion of the device fits behind the ear with the one to six or more electrodes of the device placed adjacent to (e.g., next to, within or in contact with) a targeted area of the ear (e.g., concha cymba, tragus, etc.). Right, left or two earpieces are provided in some embodiments. One or more of the vagus, auriculotemporal, trigeminal or cranial nerves may be treated in some embodiments. In certain embodiments of the present disclosure, the device stimulates the vagal nerve via contact with the concha cymba of the user's ear. The device could be unilateral or bilateral, including a single device or multiple devices connected with wires or wirelessly.

In several embodiments, dampening or blocking of nerve impulses and/or neurotransmitters are provided. In some embodiments, nerve impulses and/or neurotransmitters are enhanced. Transcutaneous neuromodulation is provided in several embodiments, although subcutaneous and percutaneous components may also be used. In some embodiments, the device includes three to six or more electrodes (e.g., 3, 4, 5, 6), and is partially implantable or is entirely transcutaneous. In some embodiments, the electrodes themselves are employed as sensing elements (e.g., for measuring nerve activity (e.g., evoked compound action potentials); for detecting electrodermal activity; or cardiac activity; or EEG) and can be placed on or proximate to a subject's wrist or placed on or proximate to a different portion of the subject's body (such as the ear, finger, portion of an arm, etc.). In some embodiments, the sensing electrode is placed on the outside of the band 36.

In some embodiments, modulation of the blood vessel (either dilation or constriction) is provided using the devices and methods described herein (e.g., through nerve stimulation). Such therapy may, in turn, reduce inflammation (including but not limited to inflammation post microbial infection). The devices and methods described herein increase, decrease or otherwise balance vasodilation and vasoconstriction through neuromodulation in some embodiments. For example, reduction of vasodilation is provided in several embodiments to treat or prevent migraine or other conditions that are aggravated by vasodilation. In other embodiments, vasoconstriction is reduced in, for example, conditions in which dilation is beneficial (such as with high blood pressure and pain). In one embodiment, reduction in inflammation treats tinnitus. In some embodiments, modulation of the blood vessel (either dilation or constriction) is used to treat tinnitus. Tinnitus may be treated according to several embodiments through modulation (e.g., stimulation) of the vagus nerve alone or in conjunction with one, two or more other nerves (including for example the trigeminal nerve, great auricular nerve, nerves of the auricular branch, auricular branch of the vagus nerve, facial nerve, the auriculotemporal nerve, etc.). In one embodiment, nerves other than the vagus nerve are modulated to treat tinnitus. Cranial/auditory nerves may be modulated to treat tinnitus and/or auricular inflammation in some embodiments. Auricular devices may be used in conjunction with devices placed on limbs to in some embodiments (e.g., an ear device along with a wrist device).

Any of the neuromodulation devices discussed herein can be utilized to modulate (e.g., stimulate) median, radial, ulnar, sural, femoral, peroneal, saphenous, tibial and/or other nerves or meridians accessible on the limbs of a subject alone or in combination with a one or more other nerves (e.g., vagal nerve) in the subject, for example, via a separate neuromodulation device. In some embodiments, provided herein are

The device 34 is detachably coupled to the band 32 for providing transcutaneous peripheral nerve stimulation to the user. In some embodiments, the band 32 is configured to be mechanically and electrically coupled to the device 34. In many embodiments, the device 34 is a wearable cuff or earpiece. The band 32 may partially or fully surround a wrist, finger, arm, leg, ankle or head. Patches may be used, but in many embodiments a patch is not used.

In some embodiments, the band 32 includes a strap 36. In some embodiments, the strap 36 secures and tightens the band 32, including the electrode system 42, to the user. In some embodiments, the band 32 is configured with a clasp or buckle that secures and tightens the band 32 on the wrist of the user.

In some embodiments, the strap 36 comprises a first portion 38 and/or a second portion 40. In some embodiments, the first and second portions 38, 40 are made from the same material. In some embodiments, the first and second portions 38, 40 are made from different materials. The materials can include silicone, urethane, a thermoplastic elastomer (TPE), fabric, or any other material. For example, in some embodiments, the first portion 38 is made from silicone and the second portion 40 is made from fabric. The first and/or second portions 38, 40 can be any color including white and can have any finish including matte. In some embodiments, the first and/or second portions 38, 40 are flexible.

The band 32 may comprise a frame 44. The frame 44 can be sized and shaped to engage with the device 34. In some embodiments, the frame 44 is manufactured by molding. In some embodiments, the frame 44 is manufactured from plastic. In some embodiments, the plastic can be any plastic such as, for example, polycarbonate (PC) and acrylonitrile butadiene styrene (ABS). In some embodiments, the frame 44 comprises any other material. The frame 44 can be any color including white and can have any finish including matte. In some embodiments, the frame 44 and the first portion 38 are manufactured as a unitary structure.

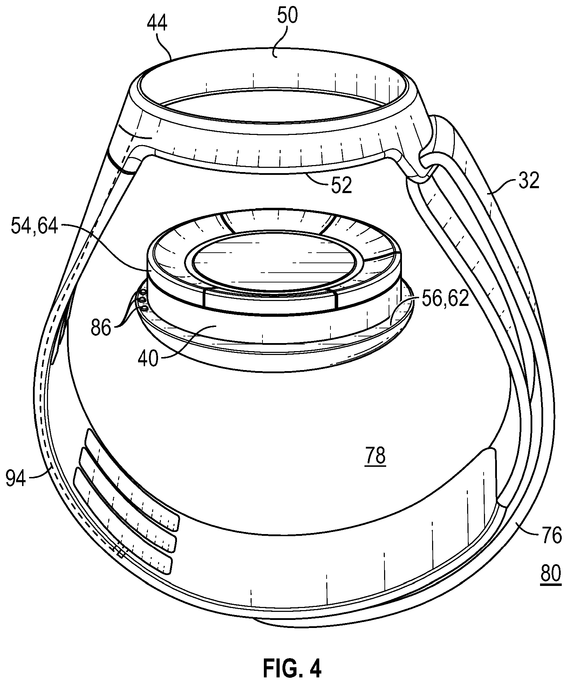

The device 34 may comprise one or more displays or screens 46 (e.g., digital displays, LEDs, etc.) to display information to the user, such as on an upper surface 58 of the device 34 (Figure 4). The screens 46 may also be touch-sensitive to receive inputs from the user. In some embodiments, the screen 46 comprises acrylonitrile butadiene styrene (ABS) or any other material. The screen 46 can be any color including light grey and can have any finish including glossy.

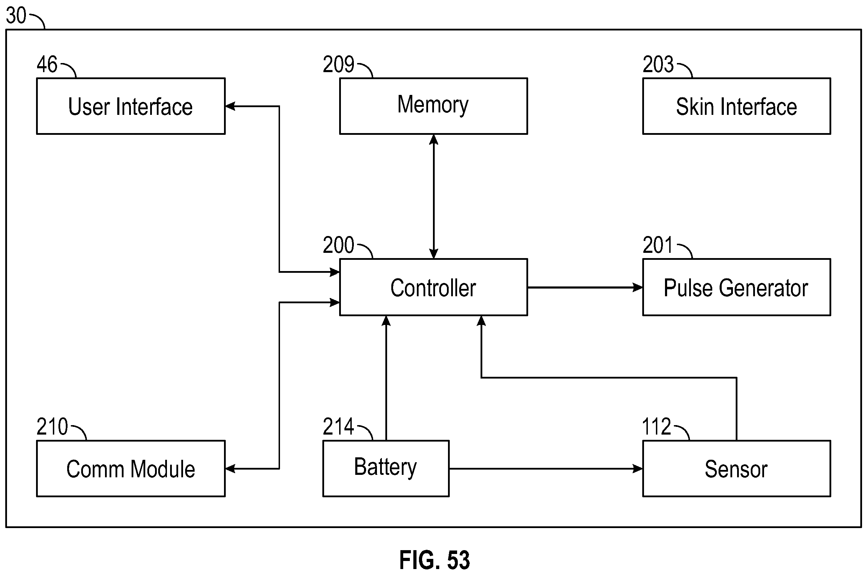

The device 34 may comprise one or more audio signal generators. In certain embodiments, the device 34 has a communication module 210 (Figure 53) to transmit data to other devices or a remote server via standard wired or wireless communication protocols. The communication module 210 may comprise one or more antennas for wireless communication over one or more communication networks. For example, the device 34 can provide wireless connectivity to the cloud for uploading and/or downloading data. In some embodiments, the device 34 connects with a hub, a base station, or other interim device via wired or wireless networks to share the data. Examples of communications networks include, but are not limited to, a local area network (LAN), a wide area network (WAN), a cellular telecommunications network, and a global network (e.g., the Internet), other network type, and any combination thereof. In some embodiments, the wireless communication includes Wi-Fi, Bluetooth, short-range radio communication, and infrared communication, among others for connecting with the hub or other interim device to share data. In some embodiments, the base station is configured for uploading and/or downloading data with the cloud via a wired or wireless communications network.

In some embodiments, the device 34 includes a Bluetooth module, a Wi-Fi module and/or a cellular module along with associated antennas. For example, in some embodiments, the device 34 comprises the cellular module, the Bluetooth module, and their associated antennas. For example, in some embodiments, the Bluetooth module and the antenna associated with the cellular module are located on the same surface within the device 34. For example, in some embodiments, the antenna for the cellular module spans across a first portion of an inner surface of the device 34 with the Bluetooth module being disposed on a second portion of the inner surface. In some embodiments, the first portion substantially surrounds the second portion. In certain embodiments, the cellular module communicates to the Internet via a mobile carrier's network. Depending on the location and carrier, various standards, such as GPRS, GSM, LTE, MIMO, and CDMA, and the like may apply.

The device 34 may also comprise a haptic motor to provide feedback or notification to the wearer by vibration. The device 34 may comprise one or more interface features, such as depressible or solid state buttons 48 for example, by which a user may interface with the device 34.

In some embodiments, the depressible or solid state buttons 48 comprise tactile electrical switches (e.g., electrically conductive dome). In some embodiments, an outer edge portion of the electrically conductive dome contacts a first electrical contact underlying the dome when the dome is in the electrically closed configuration. In some embodiments, an inner portion of the electrically conductive dome contacts a second electrical contact underlying the electrically conductive dome when the electrically conductive dome is in the electrically closed configuration. In some embodiments, the electrically conductive dome is snap reconfigurable from the electrically open configuration to the electrically closed configuration. For example, in some embodiments, a snap response occurs in response to a force applied by the tip of a finger.

**Figure 4** is a perspective side view of the system 30 with the device 34 aligned with an opening 50 in the frame 44 prior to inserting the device 34 in direction 82 into the opening 50 to secure the device 34 to the band 32. In some embodiments, the frame 44 is configured to mechanically and/or electrically interface with the device 34.

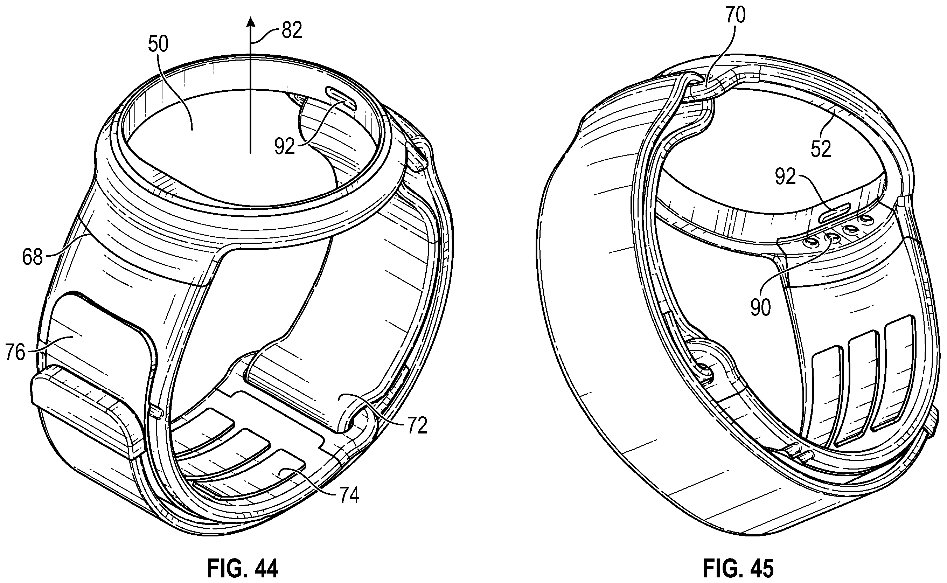

In some embodiments, the frame 44 comprises an abutment surface 52. In some embodiments, the abutment surface 52 is disposed on a surface of the frame 44. In some embodiments, one or more electrical contacts 90 (Figure 45) are disposed on the abutment surface 52. In some embodiments, the may also be treated. Devices described herein can be placed, for example, on the wrist or leg (or both) to treat limb disorders. In some embodiments, vagus nerve stimulation is used to treat restless leg syndrome, periodic limb movement disorder, repetitive movements of the limbs and/or abnormal limb sensation. With respect to the leg, a device may be placed, for example, on the thigh, calf, ankle or other location suitable to treat the target nerve(s).

In some embodiments, the device can include the ability to track a user's motion data for the purpose of gauging one, two, or more tremor frequencies of a patient. The patient could have a single tremor frequency, or in some cases multiple discrete tremor frequencies that manifest when performing different tasks. Once the tremor frequencies are observed, they can be used as one of many seminal input parameters to a personalized neuromodulation therapy.

The therapy can be delivered, e.g., transcutaneously, via one, two, or more nerves (e.g., the median and radial or ulnar nerves, and/or other nerves disclosed elsewhere herein) in order to reduce or improve a condition of the patient, including but not limited to their tremor burden. In some embodiments, the therapy modulates afferent nerves, but not efferent nerves. In some embodiments, the therapy preferentially modulates afferent nerves. In some embodiments, the therapy does not involve functional electrical stimulation.

Although transcutaneous delivery is used in many embodiments, in some embodiments at least a portion of the devices may be implanted subcutaneously or percutaneously. In one embodiment, a first electrode stimulates the median nerve, a second electrode stimulates the radial or ulnar nerve, and a third electrode stimulates the ulnar nerve. In one embodiment, two or more electrodes stimulate the same nerve (e.g., with different frequencies or other parameters). In one embodiment, one two or all of the median nerve, radial nerve, and ulnar nerve are stimulated. In some embodiments, the median nerve is modulated (e.g., stimulated) along with one, two or more other nerves in the same device or a separate device. For example, the median nerve and one or both of the radial and ulnar nerves are modulated in the same device. Optionally, another device to modulate (e.g., stimulate) in or around the ear or leg is also provided to provide synergy and may be, in one embodiment controlled by a common controller. In some embodiments, the stimulation electrodes themselves are employed as sensing elements (e.g., for measuring nerve activity (e.g., evoked compound action potentials); for detecting electrodermal activity; or cardiac activity; or EEG) and can be placed on or proximate to a subject's wrist or placed on or proximate to a different portion of the subject's body (such as the ear, finger, portion of an arm, etc.).

In several embodiments, one or more of bradykinesia, dyskinesia, gait dysfunction, dystonia and/or rigidity are treated. These may be treated in connection with Parkinson's disease or in connection with other disorders. Rehabilitation of movement is treated in some embodiments (for example to restore or improve movement and motion) in subjects who have suffered from an acute or chronic event including, for example, cardiac events (such as atrial fibrillation, hypertension, and stroke), inflammation, neuroinflammation, etc. Epilepsy is treated in one embodiment.

In some embodiments, wearable systems and methods as disclosed herein can advantageously be used to identify whether a treatment is effective in significantly reducing or preventing a medical condition, including but not limited to tremor severity. Although tremor is treated in several embodiments, the devices described herein are used to treat conditions other than tremor.

Wearable sensors can advantageously monitor, characterize, and aid in the clinical management of hand tremor as well as other medical conditions including those disclosed elsewhere herein. Clinical ratings of medical conditions, e.g., tremor severity can correlate with simultaneous measurements of wrist motion using inertial measurement units (IMUs). For example, tremor features extracted from IMUs at the wrist can provide characteristic information about tremor phenotypes that may be leveraged to improve diagnosis, prognosis, and/or therapeutic outcomes. Kinematic measures can correlate with tremor severity, and machine learning algorithms incorporated in neuromodulation systems and methods as disclosed for example herein can predict tremor severity.

In other non-tremor embodiments, physiological data including heart rate, blood glucose, blood pressure, respiration rate, body temperature, blood volume, sound pressure, photoplethysmography, electroencephalogram, electrocardiogram, blood oxygen saturation, and/or skin conductance as well as patient data from third party devices can be collected and/or aggregated to improve diagnosis, prognosis, and/or therapeutic outcomes for disorders such as migraine, depression, and/or Lyme disease. For example, physiological data including respiration rate and heart rate along with data related to sleep patterns and activity level can be collected and/or aggregated to improve diagnosis, prognosis, and/or therapeutic outcomes for depression.

In several embodiments, neuromodulation, such as neurostimulation, as used herein is used to replace pharmaceutical agents, and thus reduce undesired drug side effects. In other embodiments, neuromodulation, such as neurostimulation, is used together with (e.g., synergistically with) pharmaceutical agents to, for example, reduce the dose or duration of drug therapy, thereby reducing undesired side effects. Undesired drug side effects include for example, addiction, tolerance, dependence, GI issues, nausea, confusion, dyskinesia, altered appetite, etc.

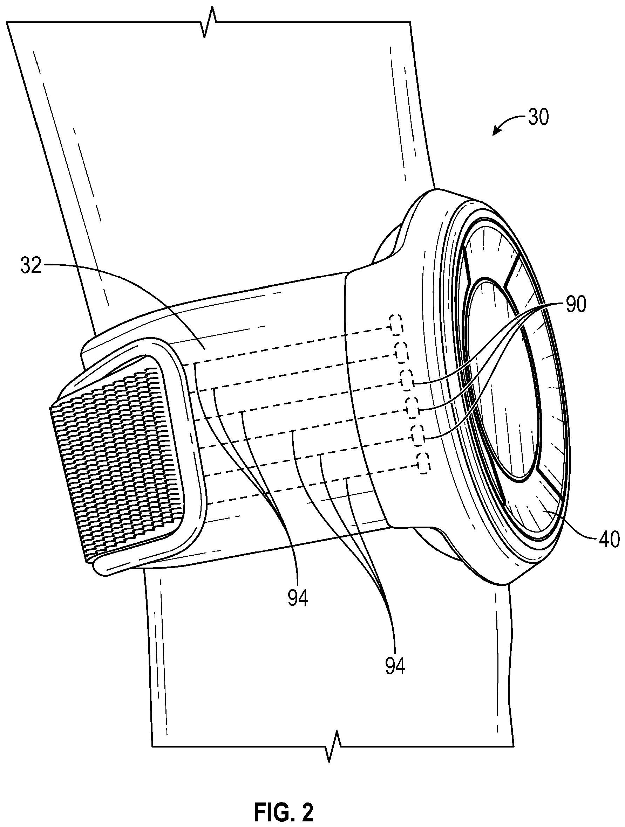

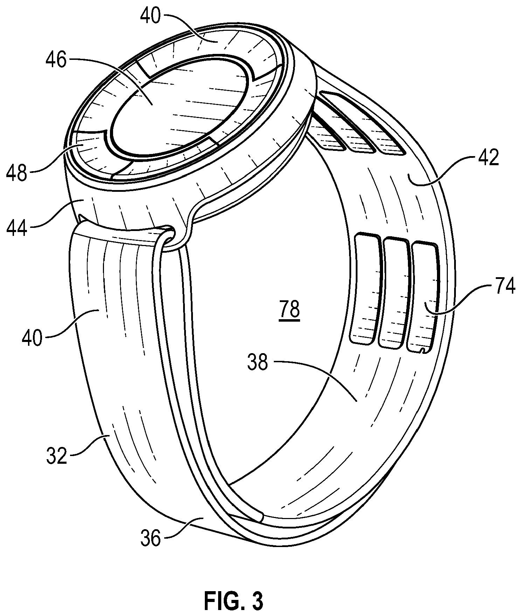

**Figure 1** is a picture of a system 30 that includes a band 32 and a device 34 that is worn by a user. **Figure 2** is another picture of the system 30 from Figure 1 taken from a side of the system 30. The band 32 may be configured to be worn by a user around his or her arm, wrist finger, leg, ankle, knee, waist, etc. In some embodiments, the band 32 comprises an electrode system 42 for distributing electrical stimulation signals generated by the device 34 to the skin of the user. **Figure 3** is a perspective view of the system 30 from Figure 1 showing the electrode system 42 on an inside 78 of the band 32.

The device 34 is detachably coupled to the band 32 for providing transcutaneous peripheral nerve stimulation to the user. In some embodiments, the band 32 is configured to be mechanically and electrically coupled to the device 34. In many embodiments, the device 34 is a wearable cuff or earpiece. The band 32 may partially or fully surround a wrist, finger, arm, leg, ankle or head. Patches may be used, but in many embodiments a patch is not used.

In some embodiments, the band 32 includes a strap 36. In some embodiments, the strap 36 secures and tightens the band 32, including the electrode system 42, to the user. In some embodiments, the band 32 is configured with a clasp or buckle that secures and tightens the band 32 on the wrist of the user.

In some embodiments, the strap 36 comprises a first portion 38 and/or a second portion 40. In some embodiments, the first and second portions 38, 40 are made from the same material. In some embodiments, the first and second portions 38, 40 are made from different materials. The materials can include, silicone, urethane, a thermoplastic elastomer (TPE), fabric, or any other material. For example, in some embodiments, the first portion 38 is made from silicone and the second portion 40 is made from fabric. The first and/or second portions 38, 40 can be any color including white and can have any finish including matte. In some embodiments, the first and/or second portions 38, 40 are flexible.

The band 32 may comprise a frame 44. The frame 44 can be sized and shaped to engage with the device 34. In some embodiments, the frame 44 is manufactured by molding. In some embodiments, the frame 44 is manufactured from plastic. In some embodiments, the plastic can be any plastic such as, for example, polycarbonate (PC) and acrylonitrile butadiene styrene (ABS). In some embodiments, the frame 44 comprises any other material. The frame 44 can be any color including white and can have any finish including matte. In some embodiments, the frame 44 and the first portion 38 are manufactured as a unitary structure.

The device 34 may comprise one or more displays or screens 46 (e.g., digital displays, LEDs, etc.) to display information to the user, such as on an upper surface 58 of the device 34 (Figure 4). The screens 46 may also be touch-sensitive to receive inputs from the user. In some embodiments, the screen 46 comprises acrylonitrile butadiene styrene (ABS) or any other material. The screen 46 can be any color including light grey and can have any finish including glossy.

The device 34 may comprise one or more audio signal generators. In certain embodiments, the device 34 has a communication module 210 (Figure 53) to transmit data to other devices or a remote server via standard wired or wireless communication protocols. The communication module 210 may comprise one or more antennas for wireless communication over one or more communication networks. For example, the device 34 can provide wireless connectivity to the cloud for uploading and/or downloading data. In some embodiments, the device 34 connects with a hub, a base station, or other interim device via wired or wireless networks to share the data. Examples of communications networks include, but are not limited to, a local area network (LAN), a wide area network (WAN), a cellular telecommunications network, and a global network (e.g., the Internet), other network type, and any combination thereof. In some embodiments, the wireless communication includes Wi-Fi, Bluetooth, short-range radio communication, and infrared communication, among others for connecting with the hub or other interim device to share data. In some embodiments, the base station is configured for uploading and/or downloading data with the cloud via a wired or wireless communications network.

In some embodiments, the device 34 includes a Bluetooth module, a Wi-Fi module and/or a cellular module along with associated antennas. For example, in some embodiments, the device 34 comprises the cellular module, the Bluetooth module, and their associated antennas. For example, in some embodiments, the Bluetooth module and the antenna associated with the cellular module are located on the same surface within the device 34. For example, in some embodiments, the antenna for the cellular module spans across a first portion of an inner surface of the device 34 with the Bluetooth module being disposed on a second portion of the inner surface. In some embodiments, the first portion substantially surrounds the second portion. In certain embodiments, the cellular module communicates to the Internet via a mobile carrier's network. Depending on the location and carrier, various standards, such as GPRS, GSM, LTE, MIMO, and CDMA, and the like may apply.

The device 34 may also comprise a haptic motor to provide feedback or notification to the wearer by vibration. The device 34 may comprise one or more interface features, such as depressible or solid state buttons 48 for example, by which a user may interface with the device 34.

In some embodiments, the depressible or solid state buttons 48 comprise a tactile electrical switches (e.g., electrically conductive dome). In some embodiments, an outer edge portion of the electrically conductive dome contacts a first electrical contact underlying the dome when the dome is in the electrically closed configuration. In some embodiments, an inner portion of the electrically conductive dome contacts a second electrical contact underlying the electrically conductive dome when the electrically conductive dome is in the electrically closed configuration. In some embodiments, the electrically conductive dome is snap reconfigurable from the electrically open configuration to the electrically closed configuration. For example, in some embodiments, a snap response occurs in response to a force applied by the tip of a finger.

**Figure 4** is a perspective side view of the system 30 with the device 34 aligned with an opening 50 in the frame 44 prior to inserting the device 34 in direction 82 into the opening 50 to secure the device 34 to the band 32. In some embodiments, the frame 44 is configured to mechanically and/or electrically interface with the device 34.

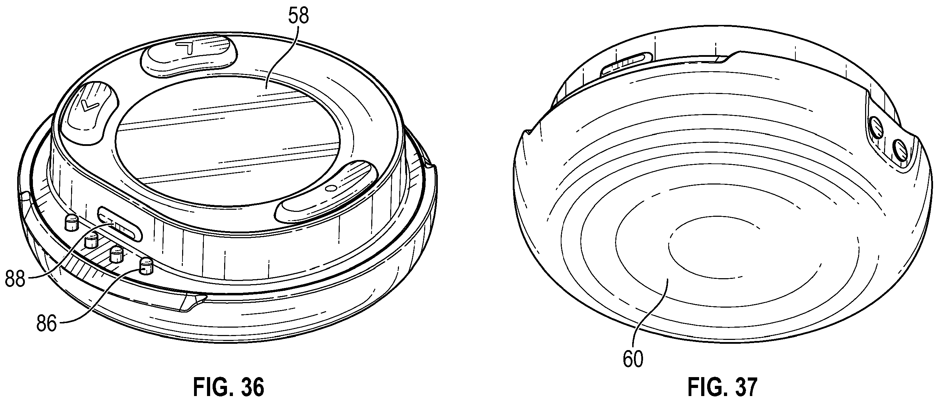

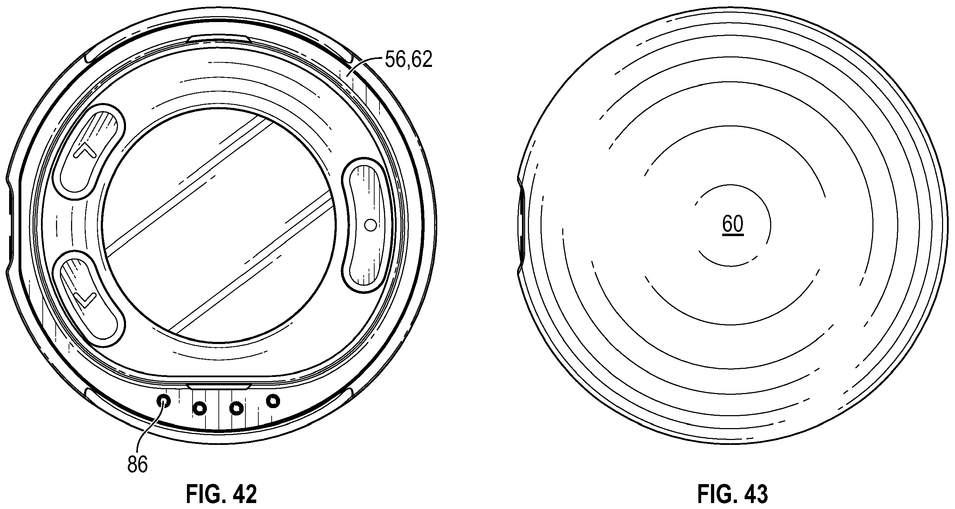

In some embodiments, the frame 44 comprises an abutment surface 52. In some embodiments, the abutment surface 52 is disposed on a surface of the frame 44. In some embodiments, one or more electrical contacts 90 (Figure 45) are disposed on the abutment surface 52. In some embodiments, the abutment surface 52 is shaped and sized to contact a contact surface 56 of the device 34 when the device 34 is secured to the band 32. At least a portion of the contact surface 55 can be located near a bottom, top, or at any location between the bottom and top of the device 34. In the illustrated embodiment, the contact surface 55 is disposed between the bottom and top of the device 34. In some embodiments, the contact surface 55 is disposed closer to the bottom of the device 34. In some embodiments, the one or more electrical contacts 90 are shaped and sized to contact one or more electrical contacts 86 (Figures 36 and 42) of the device 34 when the device 34 is secured to the band 32. In some embodiments, the one or more electrical contacts 86, 90 each comprise four contacts. In other embodiments, the one or more electrical contacts 86, 90 each comprise more or less than four contacts.

The band 32 has an outside 80 and an inside 78. The outside 80 is viewable by the user and the inside 78 faces skin of the user when the band 32 is worn by the user. In some embodiments, the device 34 is insertable from the inside 78 of the band 32 in direction 82 into the opening 50 so that the contact surface 56 abuts the abutment surface 52 of the frame 44 preventing the device 34 from passing entirely through the opening 50 and exiting the opening 50 on the outside 80 of the band 32.

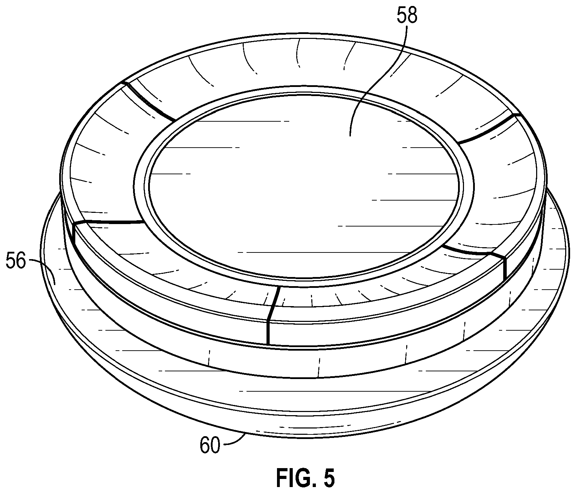

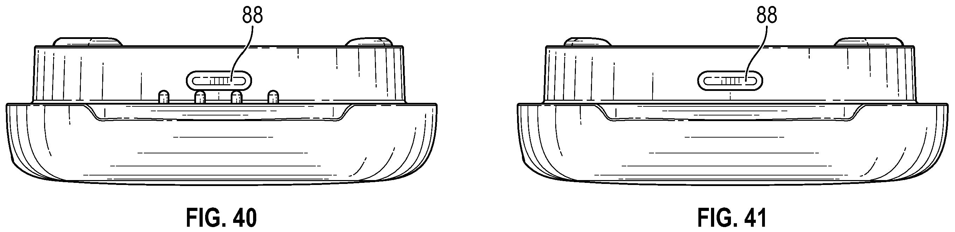

In some embodiments, the device 34 can have an upper surface 58 and a lower surface 60 (Figure 5). In some embodiments, the device 34 comprises an outer wall 54 disposed between the upper surface 58 and the lower surface 60. In some embodiments, the outer wall 54 engages the frame 44. In some embodiments, the outer wall 54 comprises one or more engagement structures 88 (Figures 40 and 41). In some embodiments, the one or more engagements structures 88 of the device 34 are positioned to engage one or more engagement structures 92 (Figures 44 and 45) of the band 32. In some embodiments, the one or more engagements structures 88 of the device 34 are formed as projections which engage with the one or more engagement structures 92 of the band which are formed as recesses. In some embodiments, the one or more engagements structures 88 of the device 34 are formed as recesses which engage with the one or more engagement structures 92 of the band which are formed as projections. In some embodiments, at least a portion of the lower surface 60 contacts a limb of the user when the system 30 is worn by the user.

In some embodiments, the band 32 captures the device 34 against the limb so that a portion of the lower surface 60 is in contact with the limb and the screen 46 is viewable by the user. In some embodiments, at least a portion of the device 34 is disposed between a surface of the band 32 and the limb. In some embodiments, at least a portion of the device 34 forms a press-fit with the band 32.

In some embodiments, the outer wall 54 is sized and shaped to be secured within the opening 50 when the device 34 is inserted in direction 82 into the opening 50. In some embodiments, the device 34 is inserted from the inside 78 of the band 32. In some embodiments, the opening 50 is sized and shaped to prevent the device 34 from passing entirely through the opening 50. In some embodiments, engagement between the one or more engagements structures 88 of the device 34 and the one or more engagement structures 92 of the band 32 inhibits inadvertent removal of the device 34 from the band 32.

The outer wall 54 can have any size or shape. In some embodiments, a portion of the outer wall 54 has a circumference that is greater than an inner circumference of the opening 50. In some embodiments, a portion of the outer wall 54 has a conical shape. For example, in some embodiments, the outer wall 54 has a tapering conical shape in a direction from the lower surface 60 to the upper surface 58. In some embodiments, the tapering conical shape prevents the device 34 from passing entirely through the frame 44 when the device 34 is captured by the band 32.

In some embodiments, the outer wall 54 has a step shape. For example, the step can comprise a riser 64 and tread 62. In some embodiments, a circumference of the riser 64 is less than an inner circumference of the opening 50. In some embodiments, the circumference of the riser 64 is not significantly greater than the inner circumference of the opening 50. For example, in some embodiments, the circumference of the riser 64 is slightly greater than the inner circumference of the opening 50 so that a press-fit (e.g., interference fit) engagement is created between the riser 64 and the opening 50. In some embodiments, the device 34 is sized and shaped so only a portion of the device 34 fits within the opening 50 when the device 34 is inserted into the frame 44 from the inside 78 of the strap 36. In some embodiments, the screen 46 is viewable within the opening 50 from the outside 80 of the strap 36.

In some embodiments, the electrical interface (e.g., the one or more electrical contacts 86) of the device 34 couples to the frame 44 via the opening 50. In some embodiments, the electrical interface of the device 34 comprises the one or more electrical contacts 86 (Figures 36 and 42). In some embodiments, the one or more electrical contacts 86 extends upward or outward from a surface of the device 34. The opening 50 may substantially match a portion of a surface of the device 34 comprising the one or more electrical contacts 86. For example, the one or more electrical contacts 86 of the device 34 can be located on a contact surface 56 while the one or more electrical contacts 90 (Figure 45) of the band 32 can be located on the frame 44 and positioned to contact the one or more electrical contacts 86.

In some embodiments, the device 34 and the frame 44 may comprise corresponding keying features which ensure the device 34 and the band 32 are coupled in an appropriate orientation.

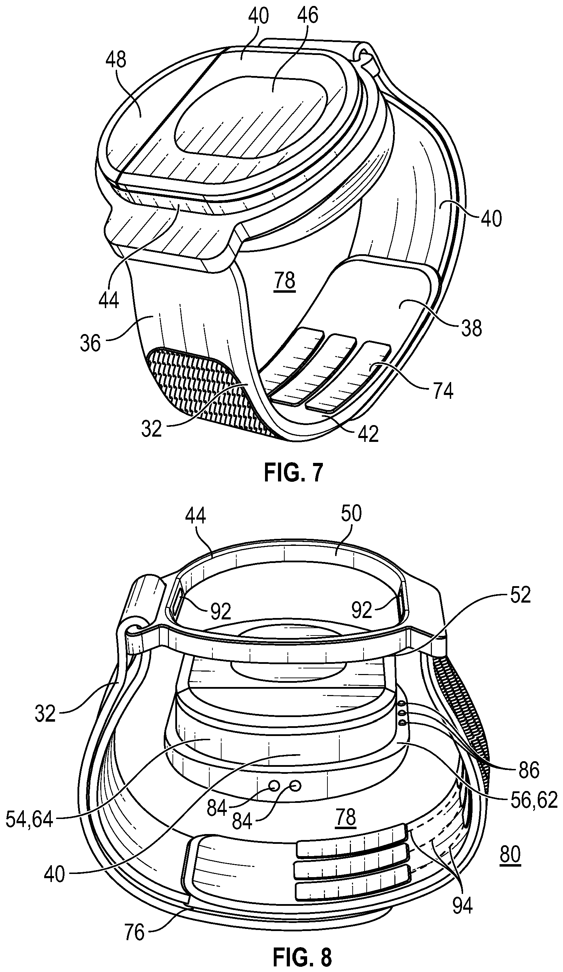

In some embodiments, the opening 50 is sized and shaped to form a receptacle that surrounds at least a portion of the outer wall 54 of the device 34. The shape of the opening 50 can match a shape of the device 34. In some embodiments, the opening 50 has an oval shape. Of course, the opening 50 need not have an oval shape and can have any other shape including the shapes illustrated in Figures 7 to 51. In some embodiments, the opening 50 has a symmetric shape across longitudinal and lateral axes of the band 32.

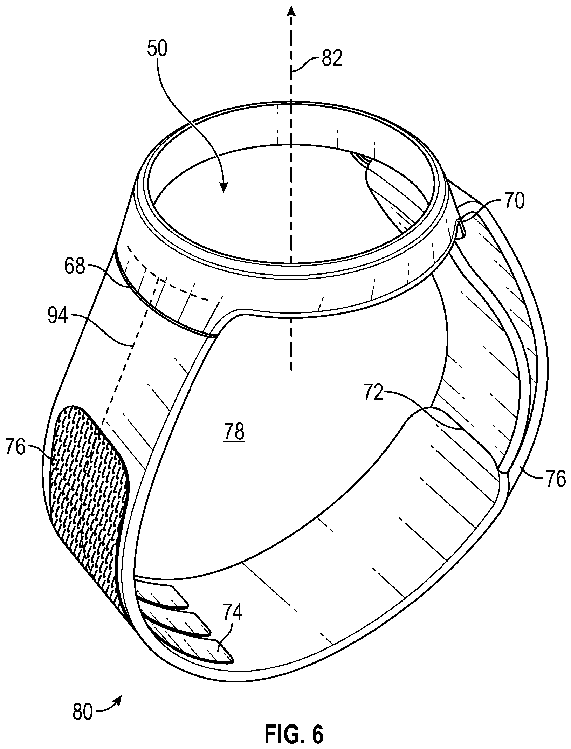

In some embodiments, a thickness or width of the frame 44 varies. For example, in some embodiments, the thickness is reduced in a region between a connection 68 and an aperture 70 (Figure 6). The variations in thickness can reduce any bending force applied by the user to remove the device 34 from the frame 44. In some embodiments, the user first holds the frame 44 at the connection 68 and the aperture 70 and then presses the screen 46 of the device 34 to pop the device 34 out of the opening 50 in a direction towards the inside 78. In this way, the frame 44 can take on a slightly curved or bent shape, such as a U-shape. In some embodiments, the frame 44 has a generally planar shape when the device 34 is secured within the opening 50. In some embodiments, the frame 44 has a slightly bent shape even when the device 34 is secured within the opening 50.

The size and shape of the connection 68 and the aperture 70 may be configured to provide one or more suitable contact surfaces for the user to manipulate the connection 68 and the aperture 70 when removing the device 34 from the frame 44. A user may use one or more of his or her fingers to pull back on the connection 68 and the aperture 70 while pushing the device 34 forward to advantageously leverage removing the device 34 from the opening 50. The connection 68 and the aperture 70 may allow the user to apply a slight bending moment to the device 34. In some embodiments, the connection 68 is adjacent to the opening 50. In some embodiments, the aperture 70 is adjacent to the opening 50.

In some embodiments where the opening 50 has a non-circular shape that matches the shape of the outer wall 54, the shape requires the user to attach the device 34 in one or more specific orientations. The keying features may ensure, for example, that one or more electrical contacts 86 of the device 34 are connected properly to the one or more electrical contacts 90 of the frame 44 and not reversed. The keying features may be particularly advantageous for embodiments where the electrical contacts form a symmetric arrangement. The keying features may ensure that the proper stimulation signal is electrically coupled to the proper electrode of the electrode system 42 and, correspondingly, the proper nerve, and prevent the device 34 from being worn on the wrong hand (e.g., right or left hand).

The shape of the opening 50, in addition to the engagement features describe below, may further prevent relative rotation of the device 34 when secured to the band 32. In this way, the device 34 may be configured (e.g., shaped and sized) to be received in the opening 50 of the frame 44. For example, the opening 50 may have any appropriate shape including those described elsewhere herein and a general height matched to a height of the outer wall 54 of the device 34. Thus, the opening 50 may be round, oval, elliptical/stadium shaped, or any other suitable shape.

The opening 50 may form a reversibly detachable interference fit or snap fit with the device 34. In some embodiments, the opening 50 may comprise the abutment surface 52 and/or a recess and the device 34 may comprise a projection. For example, the projection can be disposed on the outer wall 54 and positioned to engage with the abutment surface 52 and/or recess. Positioning the electrical contacts of the electrical interface on a surface of the frame 44 that is hidden by the device 34 may advantageously protect the electrical contacts from damage.

In some embodiments, the frame 44 may encompass all the electrical contacts with the device 34. For example, the frame 44 can include embedded conductors or wires which extend from the opening 50 (e.g., from the one or more electrical contacts 90) to the electrode system 42 in the first portion 38 of the strap 36 for the one or more electrical contacts 86 (e.g., ground and stimulation) from the device 34 to electrically contact to the electrodes 74 of the electrode system 42. The one or more electrical contacts 86 from the device 34 may be snap connections which form snap fits (e.g., annular snap fits) with corresponding one or more contacts or holes 90 on the frame 44.

In some embodiments, the one or more electrical contacts 86, 90 further include an optional return or ground contact for dispersing stimulation current from the body by returning to the stimulation source. In some embodiments, the one or more electrical contacts 86, 90 may also provide a mechanical connection between the band 32 and the device 34. In some embodiments, the one or more electrical contacts 86, 90 are metallic, electrically conductive snap fasteners to provide a mechanical connection. In some embodiments, the frame 44 may comprise recessed electrical contacts 90 and the device 34 may comprise protruding electrical contacts 86. In some embodiments, the frame 44 may comprise protruding electrical contacts 90 and the device 34 may comprise recessed electrical contacts 86.

In some embodiments, the first portion 38 of the strap comprises the electrode system 42 and is mechanically coupled to the frame 44. For example, in some embodiments, the proximal end of the electrode system 42 is engaged with the frame 44 via the connection 68. The strap 36 may be attached to the frame 44 by any suitable means, such as an adhesive, over molding, or permanent or removable mechanical fastener.

**Figure 5** is a perspective view of the device 34 from Figure 4. In some embodiments, the device 34 may have the upper surface 58 and the opposing lower surface 60. In some embodiments, the lower surface 60 is a bottom surface of the device 34. Only the upper surface 58 of the device 34 is shown in Figure 5. In some embodiments, the outer wall 54 extends from the upper surface 58 to the lower surface 60 and defines a height of the device 34. In some embodiments, at least a portion of the outer wall 54 is curved between the upper and lower surfaces 58, 60. In some embodiments, at least a portion of the outer wall 54 is flat between the upper and lower surfaces 58, 60.

The upper surface 58 and the lower surface 60 may have substantially the same shape. In some embodiments, the upper surface 58 and the lower surface 60 have different shapes. For example, the upper surface 58 and/or the lower surface 60 may be substantially rectangular, substantially oval, or an intermediate shape between a rectangle and an oval. In other embodiments, the shape may be circular, triangular, polygonal, etc. Of course, the device 34 need not have one of the enumerated shapes and can have any other shape including the shapes illustrated in **Figures 7 to 51****.**

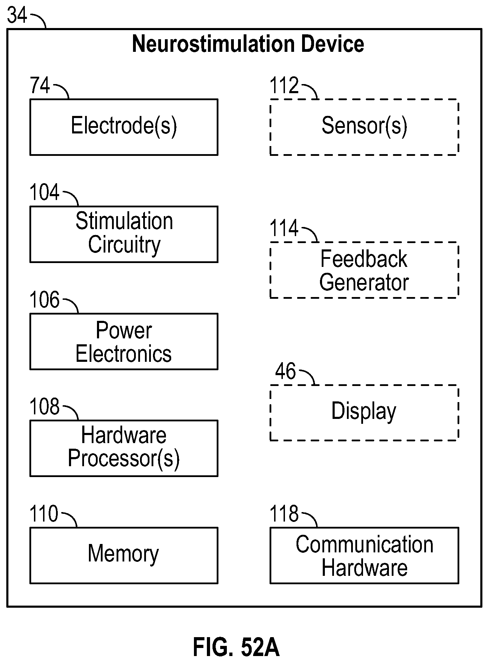

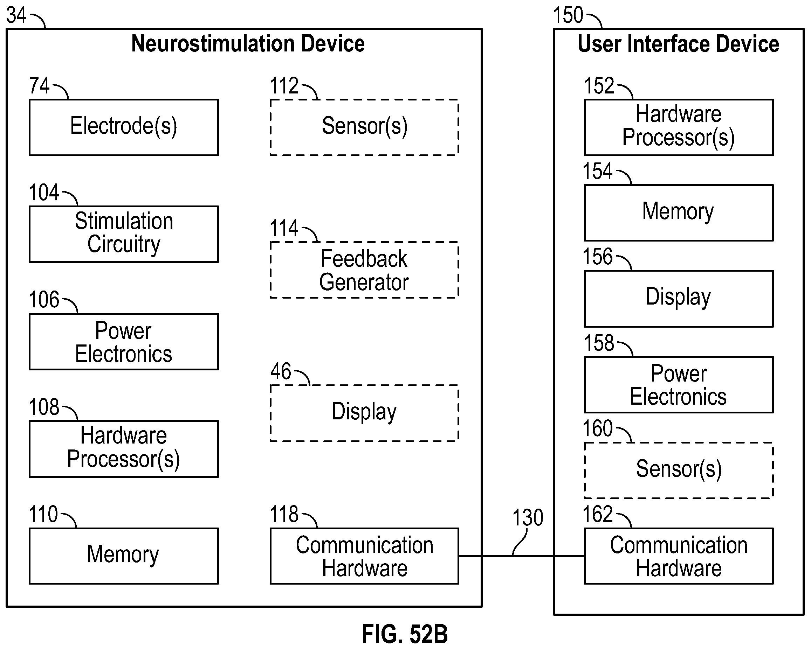

The device 34 may be configured to enclose or contain electronic circuitry for generating and providing a neurostimulation signal to be applied to the user (Figures 52A, 52B, 53). The circuitry may be self-contained in the device 34 such that the device 34 is portable. The circuitry may include a pulse generator 201 for generating an electrical stimulation pulse and a controller 200 for controlling the delivery of the electrical pulses. The device 34 may also comprise a power source, such as a battery 214. The device 34 may also contain one or more processors and memory 209. Further possible combinations of electronic circuitry for generating and providing a neurostimulation signal are described, for example, in U.S. Pat. 9,452,287 to Rosenbluth et al., U.S. Pat. No. 9,802,041 to Wong et al., PCT Pub. No. WO 2016/201366 to Wong et al., PCT Pub. No. WO 2017/132067 to Wong et al., PCT Pub. No. WO 2017/023864 to Hamner et al., PCT Pub. No. WO 2017/053847 to Hamner et al., PCT Pub. No. WO 2018/009680 to Wong et al., PCT Pub. No. WO 2018/039458 to Rosenbluth et al., PCT Pub. No. WO 2018/187241 to Hamner et al., PCT Pub. No. WO 2019/213433 to Liberatore et al., PCT Pub. No. WO 2020/006048 to Rosenbluth et al., PCT Pub. No. WO 2020/069219 to Ross et al., PCT Pub. No. WO 2020/086726 to Hamner et al., PCT Pub. No. WO 2020/185601 to Hamner et al., PCT Pub. No. WO 2021/0252278 to Hamner et al., and PCT Pub. No. WO 2021/236815 to Kent et al.

An inside, inner side, or skin side of the strap 36 can comprise the electrode system 42. The electrode system 42 may comprise the electrodes or electrical contacts 74 configured for stimulating the user. In some embodiments, the electrodes or electrical contacts 74 individually or as a subset (e.g., the electrode system 42) are removable from the strap 36. For example, in some embodiments, one or more of the electrodes or electrical contacts 74 are part of a member that is removable/replaceable from the strap 36.

The electrical contacts of the device 34 may deliver or transfer electrical signals to the electrode system 42. The electrical contacts may be positioned on the outer wall 54 of the device 34. The electrical contacts may include one electrical stimulation contact for each electrode 74 to be applied to the user. The electrical contacts may include at least one electrical stimulation contact for each nerve that is to be stimulated. For example, the electrical contacts may include an electrical stimulation contact configured to deliver a signal to the median nerve, the radial nerve, the ulnar nerve or any combination thereof. In some embodiments, stimulation may alternate between each nerve such that the nerves are not stimulated simultaneously. In some embodiments, all nerves are stimulated simultaneously. In some embodiments, stimulation is delivered to the various nerves in one of many bursting patterns. For example, the bursting patterns can include variations in stimulation parameters including, for example, on/off, time duration, intensity, pulse rate, pulse width, waveform shape, and the ramp of pulse on and off. In one embodiment the pulse rate may be from about 1 to about 5000 Hz, about 1 Hz to about 500 Hz, about 5 Hz to about 50 Hz, about 50 Hz to about 300 Hz, or about 150 Hz, and overlapping ranges therein. In some embodiments, the pulse rate may be from 1 kHz to 20 kHz. In some embodiments, a pulse width may range from, in some cases, 50 to 500 µs (micro-seconds), such as approximately 50-150,150-300, 300-500, such as 100, 200, 300, 400 µs, and overlapping ranges therein. Although frequencies below 5 kHz are used in several embodiments, some embodiments use higher frequency stimulation (e.g., of nerves at or near the wrist or ear) of 5-75 kHz (e.g., 10-40 kHz, 15-60 kHz, etc.) and a pulse width of 1-20, 10-50, 10-40 µs. The intensity of the electrical stimulation may vary from 0 mA to 500 mA (e.g., 1 mA, 2 mA, 3 mA, 4 mA, 5 mA, 6 mA, 7 mA, 8 mA, etc.), and a current may be approximately 1-11, 1-20, 5-50, 10-100 mA (e.g., 1 mA, 2 mA, 3 mA, 4 mA, 5 mA, 6 mA, 7 mA, 8 mA, etc.), and overlapping ranges therein. The electrical stimulation can be adjusted in different patients and with different methods of electrical stimulation. In some embodiments the user can adjust the current between a minimum and maximum. For example, in some embodiments, the user can adjust the current in increments (such as 0.1, 0.5, 1.0 mA increments) between 0.1-12 mA, e.g., a minimum of 0.5 mA and a maximum of 8 mA. The increment of intensity adjustment may be, for example, 0.1 mA to 1.0 mA, such as .1-.5, .5-.75, 5-1 mA, and overlapping ranges therein. In some embodiments, the stimulation may last for approximately 10 minutes to 1 hour, such as approximately 10, 20, 30, 40, 50, or 60 minutes, or ranges including any two of the foregoing values. In some embodiments, stimulation may be provided for 30, 40, 50, 60, 80, 90, 120, 150 minutes 1-4 times a day. In some embodiments, stimulation occurs for 2-15 minutes (e.g., 3, 5, 7, 10 minutes) every hour (or on another interval) for a total of 40-240 minutes (e.g., 60, 80, 90, 120, 150 minutes) in a 12 or 24 hour period. Differing dosing schedules and/or differing stimulation parameters may reduce tolerance or habituation and/or may increase patient comfort/compliance. In one embodiment, beneficial effects of stimulation are provided during off periods; for example, a patient's tremor or other symptom/indication is reduced because the prior stimulation results in a prolonged effect on the nerve(s). Thus, a patient may be able to reduce the length, duration etc. of therapy over time. In some embodiments, the parameters (e.g., frequency, amplitude, etc.) of the stimulation signal delivered to the various nerves are different between nerves. Burst patterns include but are not limited to theta burst stimulation.

Although several neurostimulation devices are described herein, in some embodiments nerves are modulated non-invasively to achieve neuro-inhibition. Neuro-inhibition can occur in a variety of ways, including but not limited to hyperpolarizing the neurons to inhibit action potentials and/or depleting neuron ion stores to inhibit firing action potentials. This can occur in some embodiments via, for example, anodal or cathodal stimulation, high frequency stimulation (e.g., greater than about 1 kHz in some cases), or continuous or intermediate burst stimulation (e.g., theta burst stimulation). In some embodiments, the wearable devices have at least one implantable portion, which may be temporary or more long term. In many embodiments, the devices are entirely wearable and non-implantable.

In some embodiments, a plurality of electrical stimuli can be delivered offset in time from each other by a predetermined fraction of multiple of a period of a measured rhythmic biological signal such as hand tremor, such as about ¼, ½, or ¾ of the period of the measured signal for example. Further possible stimulation parameters are described, for example, in U.S. Pat. 9,452,287 to Rosenbluth et al., U.S. Pat. No. 9,802,041 to Wong et al., PCT Pub. No. WO 2016/201366 to Wong et al., PCT Pub. No. WO 2017/132067 to Wong et al., PCT Pub. No. WO 2017/023864 to Hamner et al., PCT Pub. No. WO 2017/053847 to Hamner et al., PCT Pub. No. WO 2018/009680 to Wong et al., PCT Pub. No. WO 2018/039458 to Rosenbluth et al., PCT Pub. No. WO 2018/187241 to Hamner et al., PCT Pub. No. WO 2019/213433 to Liberatore et al., PCT Pub. No. WO 2020/006048 to Rosenbluth et al., PCT Pub. No. WO 2020/069219 to Ross et al., PCT Pub. No. WO 2020/086726 to Hamner et al., PCT Pub. No. WO 2020/185601 to Hamner et al., PCT Pub. No. WO 2021/0252278 to Hamner et al., and PCT Pub. No. WO 2021/236815 to Kent et al.

In some embodiments, in addition to the engagement between the opening 50 in the frame 44 and the device 34, the frame 44 can include one or more engagement structures 88, 92 for releasably securing the device 34 to the band 32. Any suitable coupling mechanism may be employed. For example, in some embodiments, the frame 44 comprises one or more hooks and/or one or more magnets. The one or more hooks can be configured to mechanical interface with a ridge or lip of the device 34 so as to secure the device 34 to the band 32 in some embodiments. A surface of the device 34 may include an aperture for the one or more hooks to enter into the device 34 and secure to the ridge or lip. Of course, the coupling mechanism may comprise a single structure in some embodiments.

In some embodiments, the one or more magnets may comprise a magnet or ferromagnetic material that is attracted to the magnet. Once the magnets are close enough that they are magnetically attracted to each other, the magnetic attraction or force facilitates keeping the device 34 and the band 32 together and/or in alignment. This engagement may provide a physical and audible confirmation to the user that the device 34 installation is complete. In some embodiments, an audible sound is heard when the device 34 is engaged with the band 32. In some embodiments, the one or more magnets facilitate coarse alignment of the opening 50 with the outer wall 54 of the device 34.

Other reversible connection mechanisms to connect the device 34 to the band 32 can be utilized as well, including but not limited to screws, rotatable/rotational connection elements, an elastomer, and the like. Non-limiting examples of such other structures include mechanical structures such as one or more holes, or recesses configured to receive protrusions, pins, Velcro^{®} (e.g., hook and loop type fastener), adhesives, or any combination of the above.

The device 34 can also include one, two, three, or more sensors 112 (Figure 52A), which can include any number of combination of inertial measurement units (IMUs) single or multi-axis accelerometers, gyroscopes, inclinometers (to measure and correct for changes in the gravity field resulting from slow changes in the device's orientation), magnetometers; fiber optic electro goniometers, optical tracking or electromagnetic tracking; electromyography (EMG) to detect firing of tremoring muscle; electroneurogram (ENG) signals; cortical recordings by techniques such as electroencephalography (EEG) or direct nerve recordings on an implant in close proximity to the nerve; heart rate or HRV sensors, galvanic skin response sensors (GSR), thermocouples, photoplethysmography sensor (PPG), temperature sensors (e.g., for body/skin temperature or ambient temperature), and/or other physiologic sensors, for example. In some embodiments, the one or more sensors 112 can be employed to measure response to therapy as well as to calibrate therapy.

The device 34, in several embodiments, can be used for the treatment of depression (including but not limited to post-partum depression, depression affiliated with neurological diseases, major depression, seasonal affective disorder, depressive disorders, etc.), inflammation, Lyme disease, stroke, neurological diseases (such as Parkinson's and Alzheimer's), and gastrointestinal issues (including those in Parkinson's disease). The devices described herein may also be used for the treatment of inflammatory bowel disease (such as Crohn's disease, colitis, and functional dyspepsia), rheumatoid arthritis, multiple sclerosis, psoriatic arthritis, osteoarthritis, psoriasis and other inflammatory diseases. The devices described herein can be used for the treatment of inflammatory skin conditions in some embodiments. The neuromodulation devices, e.g., neurostimulation devices, described herein can be used for the treatment of chronic fatigue syndrome. The devices described herein can be used for the treatment of chronic inflammatory symptoms and flare ups. Bradykinesia, dyskinesia, rigidity may also be treated according to several embodiments. In several embodiments, rehabilitation as a result of certain events are treated, for example, rehabilitation from stroke or other cardiovascular events. In several embodiments, treatment of involuntary and/or repetitive movements is provided, including but not limited to tics, twitches, etc. (including, for example, Tourette Syndrome, tic disorders). Rhythmic and non-rhythmic involuntary movements may be controlled in several embodiments. Involuntary vocal tics and other vocalizations may also be treated. Systems and methods to reduce habituation and/or tolerance to stimulation in the disorders and symptoms identified herein are provided in several embodiments by, for example, introducing variability in stimulation parameter(s) described herein.

In several embodiments, the device 34 described herein can be used for the treatment of cardiac conditions (such as atrial fibrillation, hypertension and stroke) and for the treatment of immune dysfunction. Epilepsy is treated in one embodiment. The devices described herein can be used to stimulate the autonomic nervous system. The devices described herein can be used to balance the sympathetic/parasympathetic nervous systems. Dysfunction or imbalance of the autonomic nervous system is believed to be a potential underlying mechanism for various chronic diseases. Autonomic dysfunction can develop when the nerves of the ANS are damaged or degraded or without any known neural pathology. This condition is called autonomic neuropathy or dysautonomia. Autonomic dysfunction can range from mild to life-threatening and can affect part of the ANS or the entire ANS. Sometimes the conditions that cause problems are temporary and reversible. Others are chronic, or long term, and may continue to worsen over time. Examples of chronic diseases that are associated with autonomic dysfunction include, but are not limited to, diabetes, Parkinson's disease, tremor, cardiac arrhythmias including atrial fibrillation, hypertension, overactive bladder, urinary incontinence, fecal incontinence, inflammatory bowel diseases, rheumatoid arthritis, migraine, depression, social phobia, addition, and anxiety.

In some embodiments, disorders and symptoms caused or exacerbated by microbial infections (e.g., bacteria, viruses, fungi, and parasites) are treated. Symptoms include but are not limited to sympathetic/parasympathetic imbalance, autonomic dysfunction, inflammation (including but not limited to neuroinflammation and other inflammation), motor and balance dysfunction, pain and other neurological symptoms. Disorders include but are not limited to tetanus, meningitis, Lyme disease, urinary tract infection, mononucleosis, chronic fatigue syndrome, autoimmune disorders, etc. In some embodiments, autoimmune disorders and/or pain unrelated to microbial infection is treated, including for example, inflammation (e.g., neuroinflammation, etc.), headache, back pain, joint pain and stiffness, muscle pain and tension, etc. Other disorders (e.g., hypertension, dexterity, and cardiac dysrhythmias) can also be treated using the embodiments described herein. Tourette's and other involuntary or undesired tic or movement is treated in some embodiments.

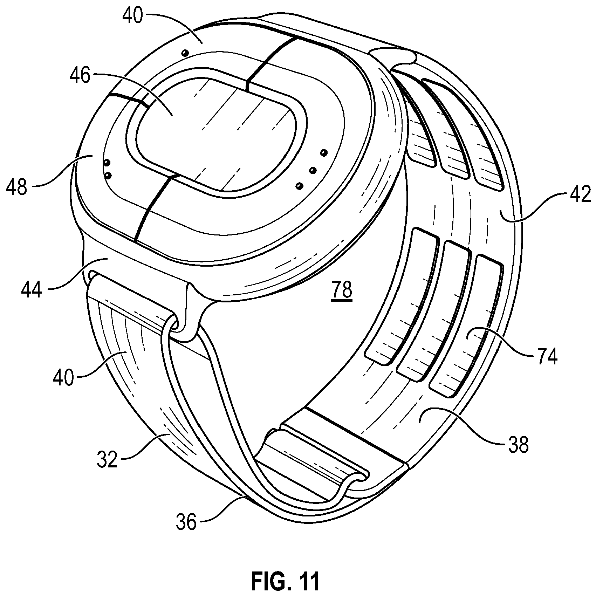

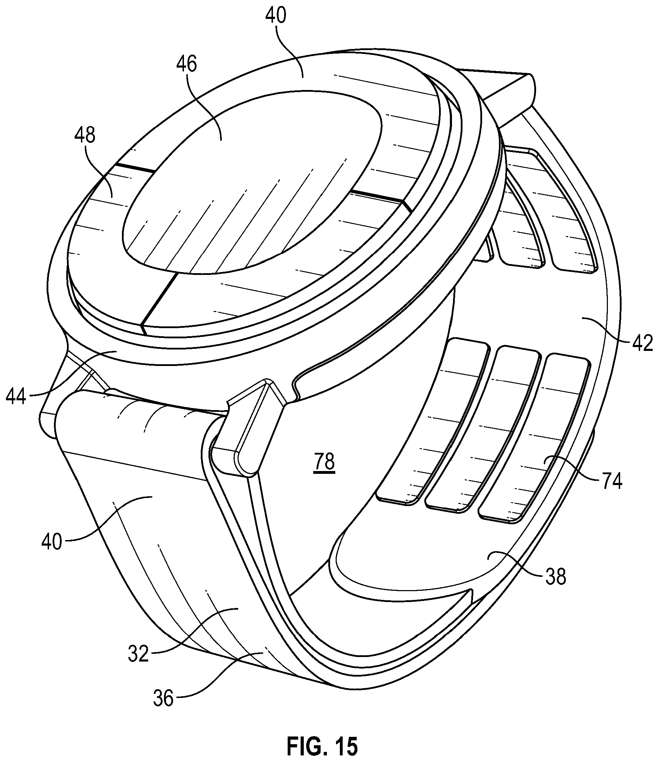

**Figure 6** is a perspective view of the band 32 from Figure 4. **Figures 7-10** **are** views similar to Figures 3-6, respectively. **Figures 11-14** **are** views similar to Figures 3-6, respectively. **Figures 15-18** **are** views similar to Figures 3-6, respectively. The band 32 can comprise the strap 36 and the frame 44. In some embodiments, the strap 36 extends from opposite sides of the frame 44 to selectively form a closed shape. In some embodiments, the strap 36 may have an adjustable length that is sufficient to accommodate any size user. In some embodiments, the strap 36 may be sized for various sizes of users (e.g., small, medium, large, child, adult, etc.). A width of the strap 36 may be less than a width of the frame 44 and/or the corresponding width of the opening 50.

The length (the longer dimension) of the frame 44 may be oriented substantially perpendicular to the length of the strap 36 and may be configured to align the length of the frame 44 with the length of the user's arm, leg, or other body appendage. The alignment of the length of the frame 44 with the length of the body part may facilitate easier movement of the body part, such as the hand and wrist, while the device 34 is being worn and may be generally less protrusive and awkward and, therefore, less likely to snag or inadvertently contact something in the user's environment. Of course, the frame 44 need not have one of the illustrated shapes and can have any other shape including the shapes illustrated in Figures 7 to 51.

In some embodiments, the strap 36 may be positioned substantially centrally along the length of the frame 44 and/or opening 50. In some embodiments, the strap 36 may be offset toward or near one side of the length of the frame 44 and/or opening 50. Offsetting the strap 36, may allow the strap 36 to be worn around, for example, the wrist of the user and the frame 44 to extend upward or proximally from the wrist in the direction of the shoulder rather than distally, or in the direction of the hand, which may beneficially allow or promote wrist movement (e.g., a larger range of motion).

In some embodiments wherein the strap 36 comprises more than one portion, the band 32 can comprise a connection 72 between the first and second portions 38, 40. In some embodiments, the connection 72 comprises over-molding the first portion 38 onto the second portion 40. For example, in some embodiments wherein the first portion 38 is manufactured from silicone and the second portion 40 is manufactured from fabric, the silicone strap portion 38 can be over-molded onto the fabric strap portion 40. In some embodiments, the connection 72 provides a secure and permanent connection between the first and second portions 38, 40. In some embodiments, the connection 72 is an aperture (e.g., a D-loop).

In some embodiments, the frame 44 can comprise a connection 68 configured to couple to a first end of the strap 36. In some embodiments, the connection 68 provides a secure and permanent connection between the frame 44 and the first portion 38 of the strap 36.

The frame 44 can also comprise an aperture 70 (e.g., a D-loop) configured to couple to a second end of the strap 36 in some embodiments. In some embodiments, the aperture 70 is configured to receive the strap 36 so as to allow the user to adjust the length of the strap 36. The effective length of the strap 36 may be adjusted by pulling the strap 36 further through the aperture 70. In some embodiments, the frame 44 and the first portion 38 of the strap 36 are manufactured as a unitary structure.

Complementary sections of hook and loop 76 fasteners (e.g., Velcro^{®}) may be attached to the strap 36 for allowing the strap 36 to form a closed loop of an adjustable length for securing the band 32 to the user, for example around the user's arm, wrist, or leg. In some embodiments, the complementary hook and loop 76 fasteners are disposed on the first and second portions 38, 40 of the strap 36. In some implementations, the band 32 may be fabricated by attaching the sections of hook and/or loop 76 fasteners to the strap 36 after the strap 36 has been received through the aperture 70.

In some embodiments, at least a portion of the strap 36 may comprise a width that is less than a maximum width of the electrode system 42. The small width portion may be configured to be received through the aperture 70. In some embodiments, one of the complementary sections of hook and loop 76 fastener is attached to a proximal portion (e.g., adjacent to the connection 68) of the strap 36 and the other section of hook and loop 76 fastener is attached to a distal portion (e.g., at the free end of the strap 36). In some embodiments, the complementary hook and loop 76 fasteners may be affixed on the same side of the strap 36. For example, the hook and loop 76 fasteners may be affixed to the outer surface of the strap 36. The free end of the strap 36 may be wrapped over itself to join the complementary hook and loop 76 fasteners together. The relative positioning of the complementary hook and loop 76 fasteners may be used to tighten or adjust the loop on the body of the user.

The electrode system 42 can have any number of electrodes 74 positioned between distal and proximal ends of the electrode system 42 for contacting the skin of the user. In some embodiments, the device includes three to six or more electrodes (e.g., 3, 4, 5, 6), and is partially implantable or is entirely transcutaneous. In some embodiments, 2-12 electrodes can be provided (e.g., 2, 3, 4, 5, 6, 7, 8, 9, 10, 11, 12, or more). In some embodiments 3-12 or more electrodes 74 are used (e.g., 3, 6, 9 or 12). In one embodiment, none of the electrodes 74 are in contact with areas that cause discomfort. The electrodes could be percutaneous or microneedle electrodes in other embodiments, or only transcutaneous (e.g., not percutaneous, microneedles, or implanted electrodes in some embodiments). In many embodiments, the transcutaneous device is a wearable band or earpiece. The band may partially or fully surround a wrist, finger, arm, leg, ankle or head. Patches may be used, but in many embodiments a patch is not used. Several embodiments provide a wrist worn or ear worn device, or both.

The electrodes 74 could be percutaneous or microneedle electrodes in other embodiments, or only transcutaneous (e.g., not percutaneous, microneedles, or implanted electrodes in some embodiments). In many embodiments, the transcutaneous device is a wearable band or earpiece. The band 32 may partially or fully surround a wrist, finger, arm, leg, ankle or head. Patches may be used, but in many embodiments a patch is not used.

In some embodiments, the electrode system 42 has a generally rectangular shape and includes six electrodes 74. In other embodiments, the electrodes 74 have a round shape or any other shape. Changing the electrode shape can also control the excitation in an area and make the stimulation more comfortable. Square or partially rounded shapes may also be provided. Although six electrodes are shown, 3-12 electrodes (e.g., 3, 9, 12 etc.) may be provided in some embodiments. In one embodiment, mechanical (e.g., vibrational) stimulation may be provided before, after or during electrical stimulation for diagnostic and/or therapeutic purposes. Such stimulation may be provided via one or more mechanical/ vibratory elements or bands configured to vibrate at a steady or varied frequencies (e.g., of between about 5-50 Hz, 4-60 Hz, 50-100 Hz, 50-300 Hz, 100-450 Hz and overlapping ranges therein). Likewise, the electrical stimulation parameters disclosed herein can be varied or steady within a given time frame (seconds, minutes, hours, etc.). Single or multiple frequencies can be used (e.g., two, three or more electrical stimulations and/or mechanical/vibrational stimulations) at the same, overlapping or different nerves. In one embodiment, varying frequency or other parameters reduces tolerance or habituation and/or increases patient comfort/compliance.

In some embodiments that have six electrodes 74, the six electrodes 74 can be arranged in two sets of three electrodes 74 spaced along the length of the first portion 38 of the band 32. Of course, the electrode system 42 is not limited to the illustrated shape or number of electrodes 74. In some implementations, the electrode system 42 of the band 32 may be fabricated as a single flat piece of flexible material. Fabricating this portion as a single piece (e.g., first portion 38) of material may simplify the manufacturing process.

As described elsewhere herein, in some embodiments, there may be one or more electrodes 74 for each electrical contact 90. The electrodes 74 can electrically connect to the complementary electrical contacts 90 on the frame 44 using one or more electronic traces 94 when the device 34 is secured to the frame 44. In some embodiments, the one or more electronic traces 94 are embedded within the strap 36 (see Figure 2). In some embodiments, the one or more electronic traces 94 are embedded within the first portion 38 of the strap 36. In some embodiments, a subset of the electrodes 74 electrically connects to a subset of the one or more traces 94. The electrical contacts 90 can include stimulation contacts and/or ground contacts. In some embodiments, the electrodes 74 may be spatially arranged in the same manner as the electrical contacts 90. In some embodiments, the electrodes 74 may be arranged differently. For example, the electrodes 74 may be arranged such that the electrodes 74 are positioned, either axially and/or at least partially around a circumference of a body part (e.g., a wrist). In some embodiments, the electrodes 74 may be configured to be generally in-line with the axon(s) of the target nerve being stimulated.

In some embodiments, the electrode system 42 employs three or more electrodes 74 to apply a stimulation signal to the patient. For example, in some embodiments, at least one electrode 74 is redundant to another electrode (e.g., 2 or more redundant common electrodes and/or 2 or more redundant stimulation electrodes). In this way, even if the electrical contact between one of the two electrodes 74 and the patient's skin is poor increasing resistance, the electrical contact between the redundant electrode 74 and the patient's skin can complete the electrical circuit with a normal or expected level of resistance.

In some embodiments, the two or more common electrodes 74 and/or 2 or more stimulation electrodes 74 are circumferentially spaced about the band 32 so that even if the band 32 rotates slightly on the wrist causing an electrode 74 to lose contact with the patient's skin, the redundant electrode 74 will still be in contact with the patient's skin to compete the circuit with a normal or expected level of resistance. In this way, the desired stimulation signal (e.g., frequency, phase, timing, amplitude, and/or offsets) is applied to the patient even when the band 32 rotates on the patient's wrist. The band 32 is less sensitive to electrical contact variations between the electrodes 74 and the patient's skin caused by variations in the angular orientation of the band 32 on the wrist.

In some embodiments, the components of the electrode system 42 can be integrated into the band 32. The advantage of this construction where the electrical contacts are on the device 34 is that electronics are not needed in the band 32.

The one or more electrodes 74 are configured to contact skin of the user. In some embodiments, an internal layer of the electrode 74 is formed by over molding the one or more electrodes 74 with a conductive material (e.g., silicone). In this way, surfaces of the internal layer expose the one or more electrodes 74.

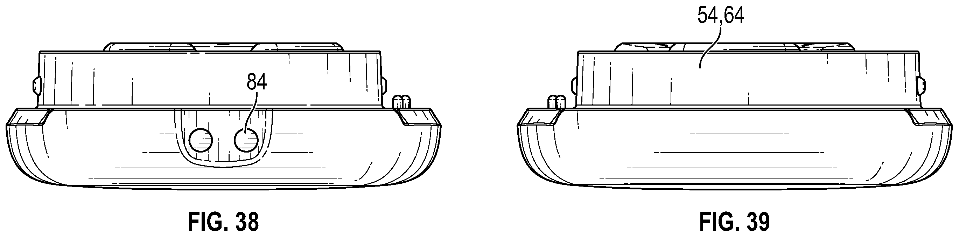

In some embodiments, the system 30 comprises one or more electrical connectors 84 (Figure 38). In the illustrated embodiment, the one or more electrical connectors 84 are disposed so as to be accessible for electrically charging the system 30. In some embodiments, the one or more electrical connectors 84 are disposed on the device 34. In some embodiments, the one or more electrical connectors 84 connect the system 30 to complementary electrical connectors on the base station (not shown). The base station can be configured to stream movement sensor and usage data on a periodic basis, e.g., daily and charge the neurostimulation device 34. In some embodiments, the base station can be connected to the cloud or internet via any wired or wireless connection protocol and configured for uploading and/or downloading data with the cloud. In some embodiments, the base station and the neurostimulation device 34 share data when the neurostimulation device 34 is being charged by the base station. In some embodiments, the base station and the neurostimulation device 34 wirelessly transmit and receive data. In some embodiments, the controller 200 can consistently update the neurostimulation device 34 to provide a more tailored therapeutic experience to the user. The base station is used, in some embodiments, to both charge and sync data.

In some embodiments, the user is able to review their tremor improvement scores (e.g., kinematic data and/or patient satisfaction ratings) via a user portal during and/or after an assessment period (e.g., one or more therapy sessions). The user, via the user portal, can select the waveform pattern for future therapy sessions based on their tremor improvement scores. In some embodiments, the waveform can be selected based on which waveform maximizes tremor improvement scores and/or other criteria (e.g., patient satisfaction), thereby providing therapy recommendations. In some embodiments, the base station downloads the selected waveform from the cloud and then transfers the waveform to the neurostimulation device 34 for future therapy sessions.

In some embodiments, the wearable system 30 transcutaneously delivers electrical signals to one or more nerves of the user. In some embodiment, the device 34 is configured as a durable component 34. In some embodiments, the band 32 is configured as a replaceable component 32. In some embodiments, the replaceable component 32 at least partially encircles a limb of the user. In some embodiments, the replaceable component 32 comprises the frame 44. In some embodiments, the frame 44 contacts the durable component 34 when the replaceable component 32 maintains the durable component 34 in contact with the skin of the user. In some embodiments, the frame 44 comprises a receptacle. In some embodiments, the receptacle is sized and shaped to receive at least a portion of the durable component 34.

In some embodiments, the durable component 34 is able to withstand more use than the replaceable component 32. In some embodiments, the durable component 34 has a useful life greater than a useful life of the replaceable component 32. In some embodiments, the useful life of the replaceable component 32 may range from, in some cases, 30 to 210 days, such as approximately 30 to 50, 50 to 70, 70 to 90, 90 to 110, 110 to 130, 130 to 150, 150 to 170, 170 to 190, 190 to 210, such as 40, 60, 80, 100, 120, 140, 160, 180, 200, and overlapping ranges therein. In some embodiments, the durable component 34 ceases operation at the end of the useful life of the replaceable component 32. In some embodiments, the durable component 34 provides a warning to the user at a predetermined amount before the end of the useful life of the replaceable component 32 to replace the replaceable component 32. In some embodiments, the durable component 34 provides one or more warnings to the user 20 days, 15 days, 10 days, 5 days, and/or 1 day before the end of the useful life of the replaceable component 32. In some embodiments, the warning is displayed on the screen or display 46.

In some embodiments, the useful life of the replaceable component 32 is predetermined. In some embodiments, the useful life is determined dynamically based on one or more characteristics. In some embodiments, the one or more characteristics can include, for example, efficacy of treatment, a change in impedance over time between the electrodes and the skin of the user, wear and tear on the electrodes, build-up of material on the electrode surface, cumulative stimulation time, geographic location, characteristics of the user, cumulative intensity of the stimulation, and/or age of the replaceable component 32. In some embodiments, an automatic shut off is implemented after 90 days of use of a replaceable component or if the system detects a malfunction, with optional warnings provided prior to said shut off.

In some embodiments, the durable component 34 comprises at least one electrode 74. In some embodiments, the replaceable component 32 is configured to maintain the durable component 34 and the at least one electrode 74 in contact with skin of the user by applying a force to the durable component 34 in a direction towards the skin. In some embodiments, the direction of the force is perpendicular to the skin of the user.

In some embodiments, the durable component 34 comprises a screen 46. In some embodiments, the screen 46 is visible to the user when the durable component 34 is in contact with the skin of the user.

In some embodiments, the at least one electrode 74 contacts the skin of the user at a location different than where the durable component 34 contacts the skin of the patient. In some embodiments, the at least one electrode 74 comprises a first electrode 74 and a second electrode 74. In some embodiments, the first electrode 74 is configured to stimulate the median nerve of the user and the second electrode 74 is configured to stimulate the radial or ulnar nerve of the user. In some embodiments, the at least one electrode 74 comprises a return or ground electrode 74 configured to be electrically coupled to the user.

In some embodiments, the system 30 comprises an electrical contact or coupling 86, 90 for conducting electrical signals between the replaceable component 32 and the durable component 34. In some embodiments, the electrical contact or coupling 86, 90 comprises an electrical interconnect. In some embodiments, the electrical interconnect is spring-loaded. In some embodiments, the electrical interconnect moves from a retracted position to an extended position when the durable component 34 is removed from the replaceable component 32.

In some embodiments, the system 30 comprises one or more engagement structures or mechanical couplings 88, 92 for inhibiting separation of the replaceable component 32 from the durable component 34 in the absence of a force. In some embodiments, the mechanical coupling 88, 92 comprises an abutment surface. In some embodiments, the mechanical coupling 88, 92 comprises a contact surface. In some embodiments, the mechanical coupling 88, 92 comprises the opening 50. In some embodiments, at least a portion of the durable component 34 forms a press-fit with the disposable component 32. In some embodiments, a magnitude of a force applied by the user to remove the durable component 34 from the disposable component 32 is less than a magnitude of the force applied by the disposable component 32 to maintain the durable component 34 in contact with the skin of the user. In some embodiments, a direction of the force which removes the durable component 34 from the disposable component 32 is parallel to a direction of the force which maintains the durable component 34 in contact with the skin of the user.

In some embodiments, the disposable component 32 comprises the first portion 38 coupled to the second portion 40. In some embodiments, the first portion 38 is manufactured from silicone and the second portion 40 is manufactured from fabric. In some embodiments, the disposable component 32 is flexible.

In some embodiments, the disposable component 32 comprises the electrode system 42. In some embodiments, the electrode system 42 comprises an inner side and an outer side. In some embodiments, the inner side comprises the at least one electrode 74. In some embodiments, the outer side can include the at least one electrode 74. For example, an electrode 74 disposed on the outer side can be used as a sensor to contact a selected location on the user's body. In some embodiments, the disposable component 32 is configured to be tightened about a limb of the user. In some embodiments, tightening the disposable component 32 forces the at least one electrode 74 firmly against the skin of the user. In some embodiments, the electrode system 42 comprises one or more electrical traces 94. In some embodiments, the one or more electrical traces 94 are in electrical contact with the at least one electrode 74. In some embodiments, the one or more electrical traces 94 are in electrical contact with the durable component 34 at least when the replaceable component 32 is maintaining the durable component 34 in contact with the skin of the user.

In some embodiments, the durable component 34 is a neurostimulation device. In some embodiments, the electrical signals delivered to the one or more nerves of the user block nerve signals. In some embodiments, the electrical signals delivered to the one or more nerves of the user stimulate nerve signals.

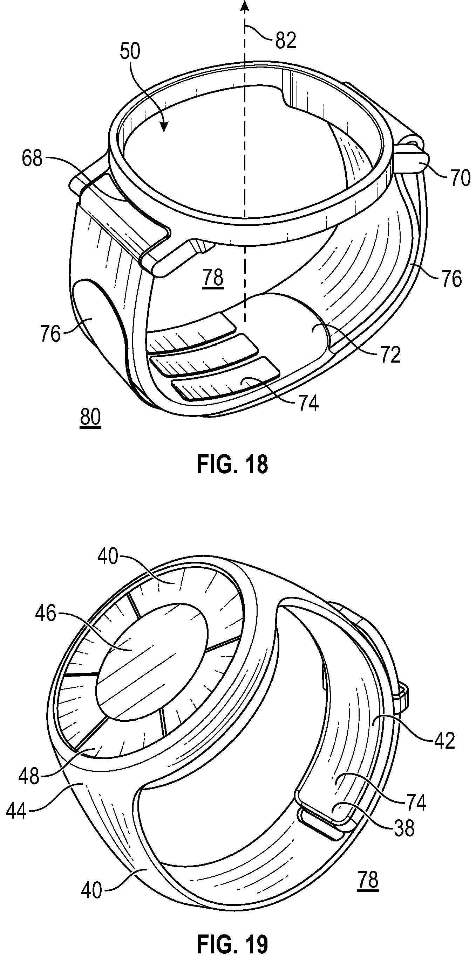

**Figure 19** is a perspective view of a system 30 similar to the system 30 of Figure 1 showing the electrode system 42 on an inside 78 of the band 32. The device 34 is detachably coupled to the band 32 for providing transcutaneous peripheral nerve stimulation to the user. In some embodiments, the band 32 is configured to be mechanically and electrically coupled to the device 34. In many embodiments, the device 34 is a wearable cuff or earpiece. The band 32 may partially or fully surround a wrist, finger, arm, leg, ankle or head. Patches may be used, but in many embodiments a patch is not used.

In some embodiments, the band 32 includes a strap 36. In some embodiments, the strap 36 secures and tightens the band 32, including the electrode system 42, to the user. In some embodiments, the band 32 is configured with a clasp or buckle that secures and tightens the band 32 on the wrist of the user. The band 32 may comprise a frame 44. The frame 44 can be sized and shaped to engage with the device 34.

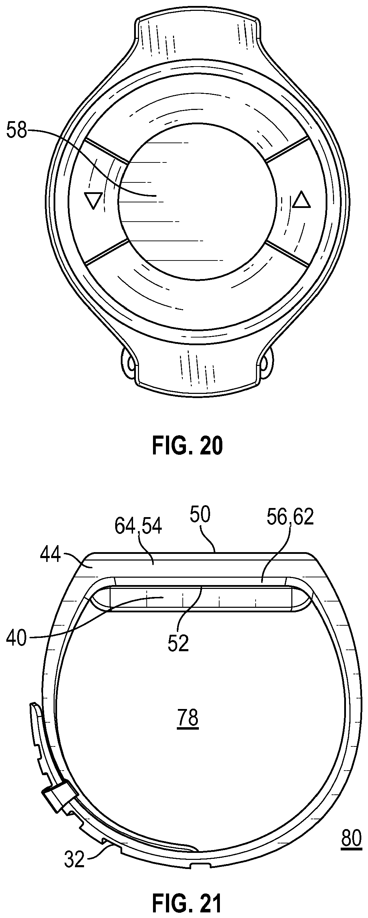

**Figure 20** is a top plan view of the system 30 from Figure 19. **Figure 21** is a side view of the system 30 from Figure 19. In some embodiments, the frame 44 is configured to mechanically and/or electrically interface with the device 34. In some embodiments, the frame 44 comprises an abutment surface 52. In some embodiments, the abutment surface 52 is disposed on a surface of the frame 44. In some embodiments, one or more electrical contacts 90 are disposed on the abutment surface 52. In some embodiments, the abutment surface 52 is shaped and sized to contact a contact surface 56 of the device 34 when the device 34 is secured to the band 32. In some embodiments, the one or more electrical contacts 90 are shaped and sized to contact one or more electrical contacts 86 of the device 34 when the device 34 is secured to the band 32. In some embodiments, the one or more electrical contacts 86, 90 each comprise four contacts. In other embodiments, the one or more electrical contacts 86, 90 each comprise more or less than four contacts.

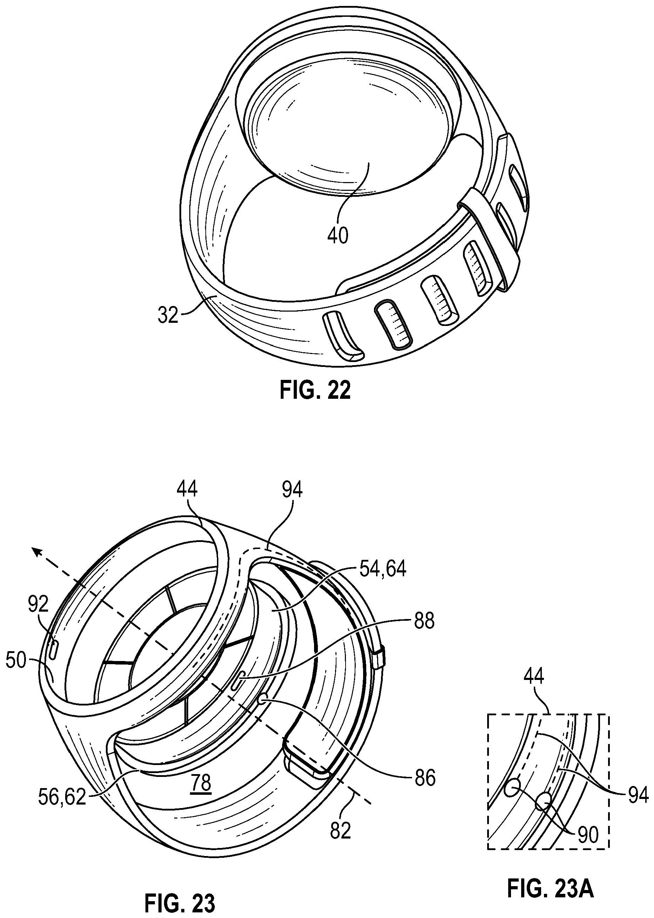

**Figure 22** is a bottom perspective of the system from Figure 19. **Figure 23** is a perspective top, side view of the system 30 with the device 34 aligned with an opening 50 in the frame 44 prior to inserting the device 34 in direction 82 into the opening 50 to secure the device 34 to the band 32. **Figure 23A** is a partial view of the frame 44 from Figure 23 showing one or more electrical contacts 90. The band 32 can comprise the strap 36 and the frame 44. In some embodiments, the strap 36 extends from opposite sides of the frame 44 to selectively form a closed shape. In some embodiments, the strap 36 may have an adjustable length that is sufficient to accommodate any size user. In some embodiments, the strap 36 may be sized for various sizes of users (e.g., small, medium, large, child, adult, etc.). A width of the strap 36 may be less than a width of the frame 44 and/or the corresponding width of the opening 50.

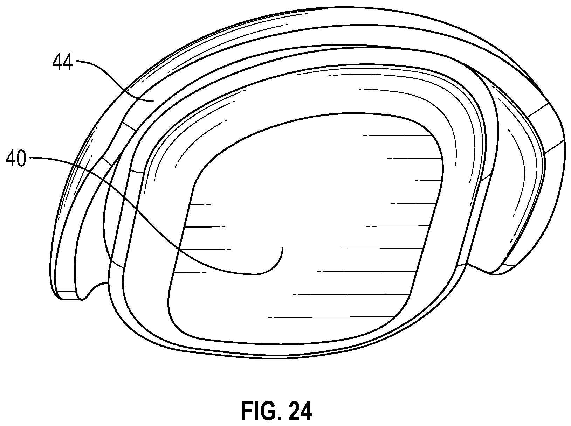

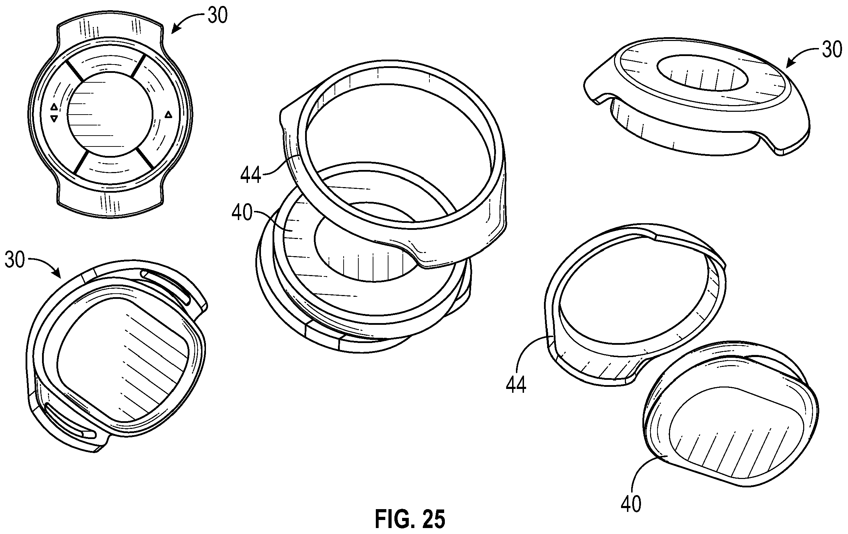

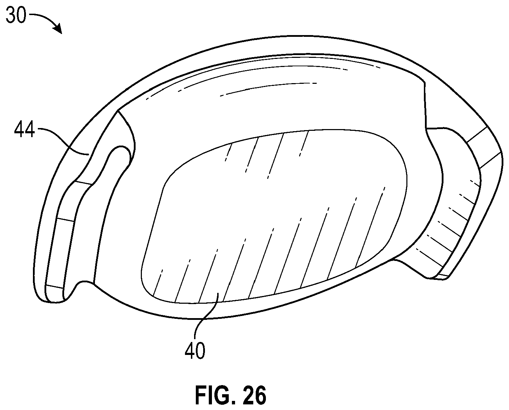

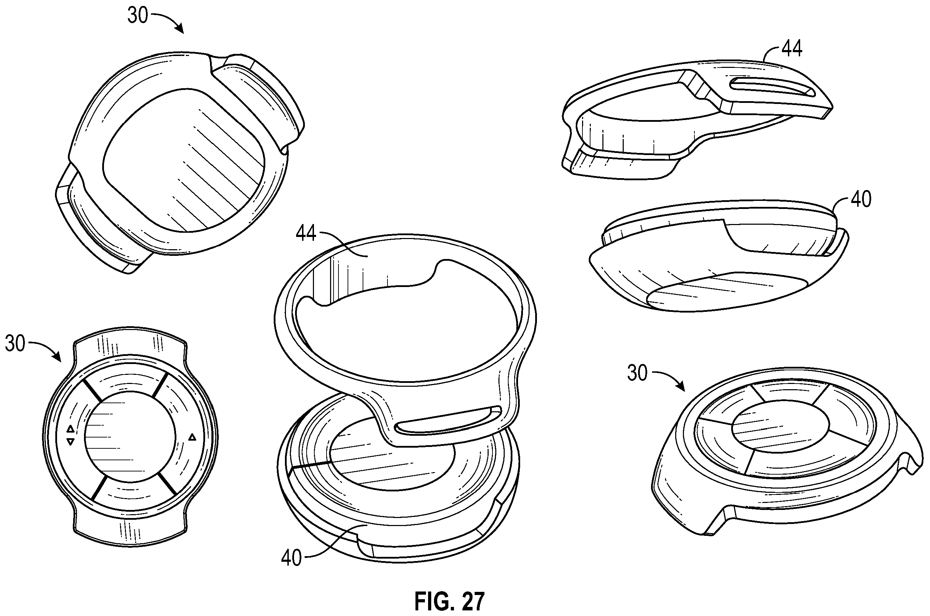

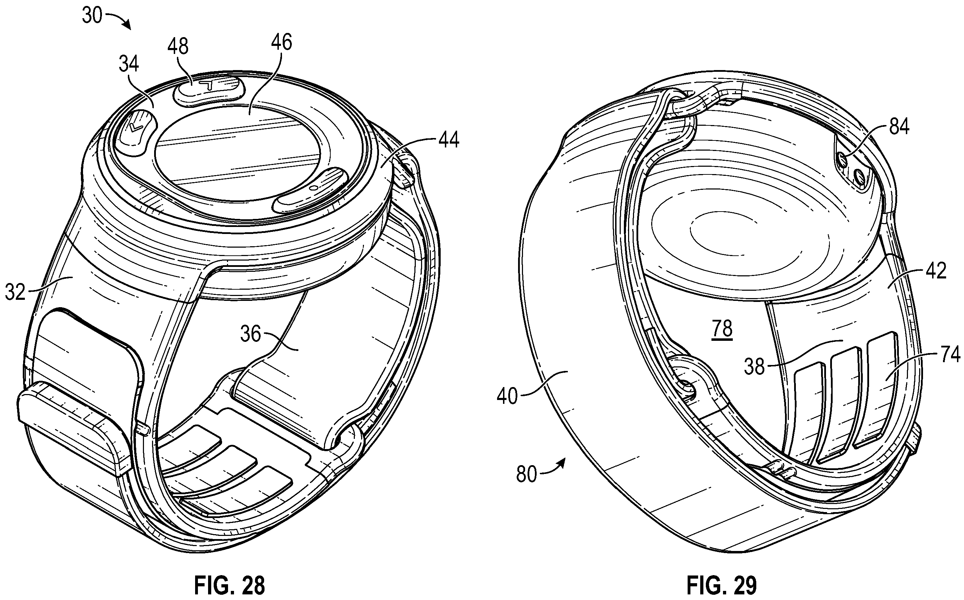

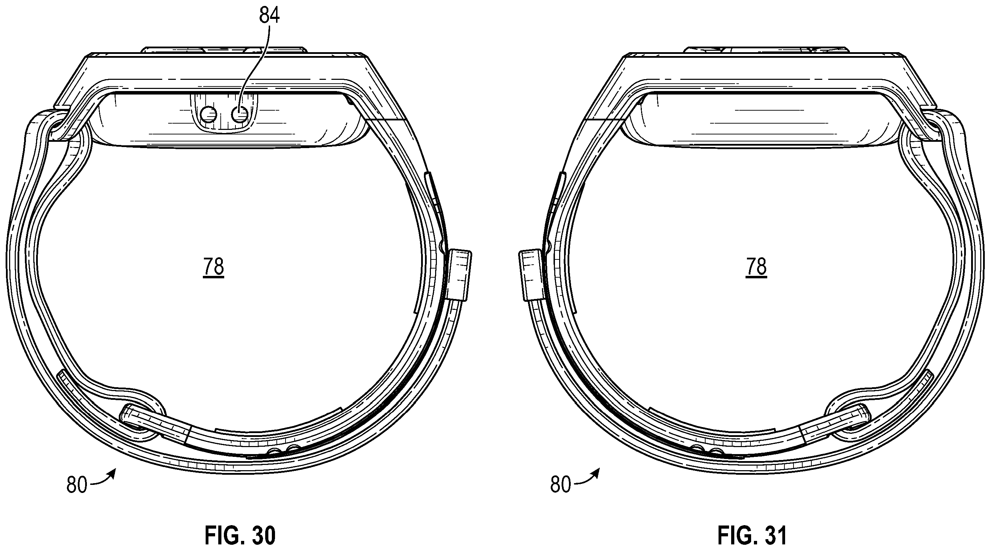

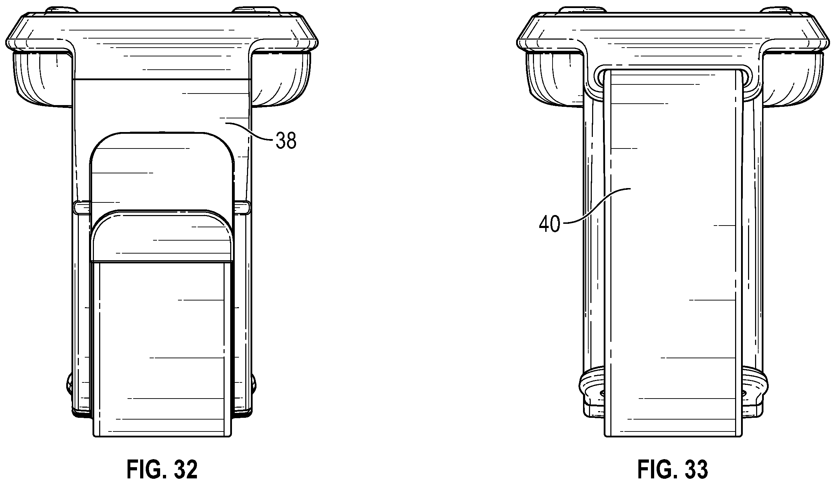

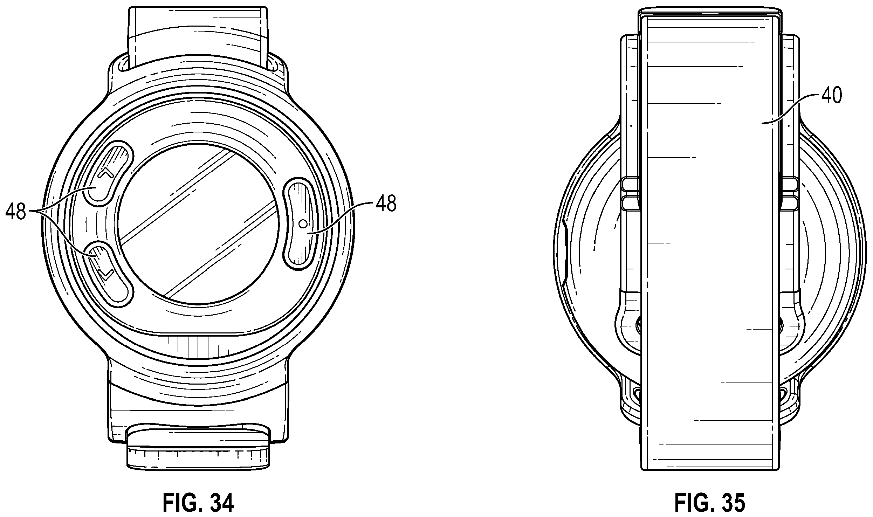

The system 30 can comprise any combination of features disclosed in any of the figures. For example, Figure 24 is a bottom perspective view of the system 30 with the band 32 removed. Figure 25 includes views of the system 30 in Figure 24. Figure 26 is another bottom perspective view of the system 30 with the band 32 removed. Figure 27 includes views of the system 30 in Figure 26. Figure 28 is a front-right perspective view of a system 30 similar to Figures 1-6. Figure 29 is a rear-left perspective view of the system 30 of Figure 28. Figure 30 is a rear elevational view of the system 30 of Figure 28. Figure 31 is a front elevational view of the system 30 of Figure 28. Figure 32 is a right side elevational view of the system 30 of Figure 28. Figure 33 is a left side elevational view of the system 30 of Figure 28. Figure 34 is a top plan view of the system 30 of Figure 28. Figure 35 is a bottom plan view of the system 30 of Figure 28.

**Figure 36** is a front-right perspective view of a device 34 from Figure 28. Figure 37 is a rear-left perspective view of the device 34 from Figure 28. Figure 38 is a rear elevational view of the device 34 from Figure 28. Figure 39 is a front elevational view of the device 34 from Figure 28. Figure 40 is a right side elevational view of the device 34 from Figure 28. Figure 41 is a left side elevational view of the device 34 from Figure 28. Figure 42 is a top plan view of the device 34 from Figure 28. Figure 43 is a bottom plan view of the device 34 from Figure 28.

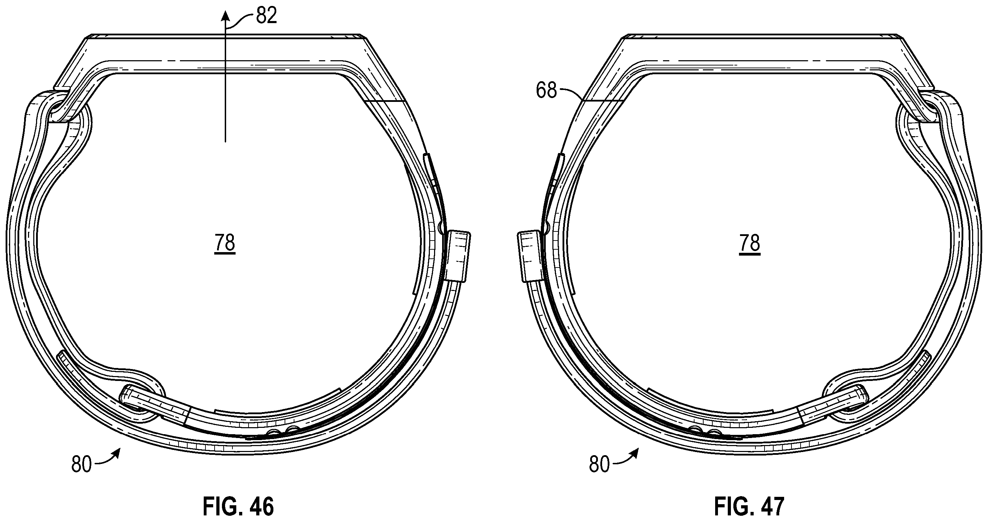

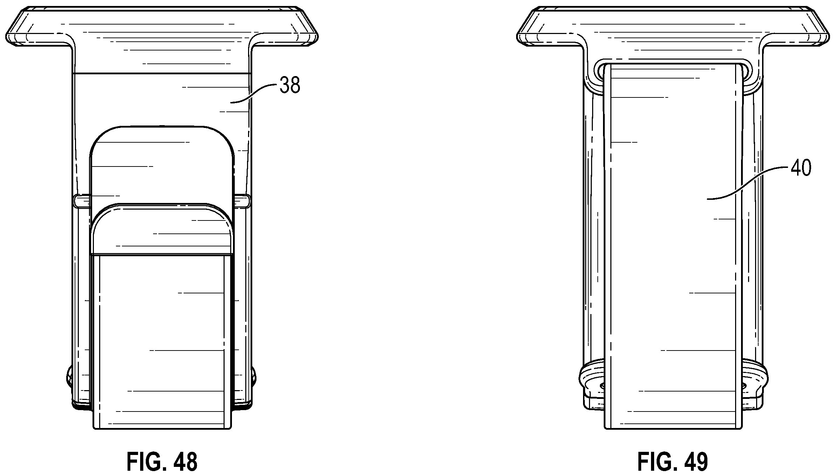

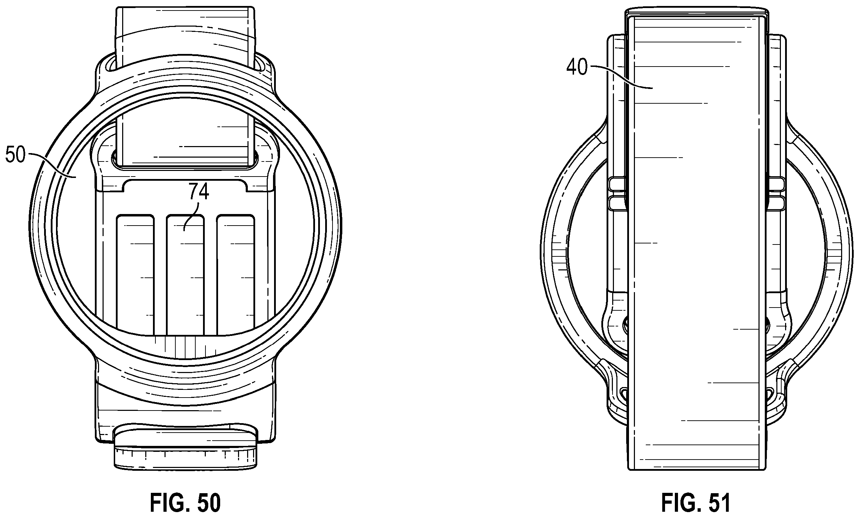

**Figure 44** is a front-right perspective view of a band 32 from Figure 28. Figure 45 is a rear-left perspective view of the band 32 from Figure 28. Figure 46 is a rear elevational view of the band 32 from Figure 28. Figure 47 is a front elevational view of the band 32 from Figure 28. Figure 48 is a right side elevational view of the band 32 from Figure 28. Figure 49 is a left side elevational view of the band 32 from Figure 28. Figure 50 is a top plan view of the band 32 from Figure 28. Figure 51 is a bottom plan view of the band 32 from Figure 28. Accordingly, the system 30 can have any number of different configurations. Thus, while certain combinations of features are illustrated in each figure, the features are not limited to only being incorporated as part of the illustrated combinations. In this way, any of the features disclosed in any of the figures can be employed with any other feature disclosed in any of the figures. Accordingly, any of the features illustrated in **Figures 1 to 54E** can be combined in any way.

Figure 52A illustrates a block diagram of an example neuromodulation (e.g., neurostimulation) device 34. The device 34 includes multiple hardware components which are capable of or programmed to provide therapy across the skin of the user. As illustrated in Figure 52A, some of these hardware components may be optional as indicated by dashed blocks. In some instances, the device 34 may only include the hardware components that are required for stimulation therapy. The hardware components are described in more detail herein.

The device 34 can further include stimulation circuitry 104 for generating signals that are applied through the electrode(s) 74. In certain embodiments, the signals can vary in, for example, frequency, phase, timing, amplitude, on/off cycling, or offsets. The device 34 can also include power electronics 106 for providing power to the hardware components. For example, the power electronics 106 can include a battery.

The signals can vary in frequency, phase, timing, amplitude, or offsets. The device 34 can also include power electronics 106 for providing power to the hardware components. For example, the power electronics 106 can include a battery.

The device 34 can include one or more hardware processors 108. The hardware processors 108 can include microcontrollers, digital signal processors, application specific integrated circuit (ASIC), a field programmable gate array (FPGA) or other programmable logic device, discrete gate or transistor logic, discrete hardware components, or any combination thereof designed to perform the functions described herein. In an embodiment, all of the processing discussed herein is performed by the hardware processor(s) 108. The memory 110 can store data specific to patient and rules as discussed below.

In some embodiments, a tremor signal can be calculated based on input from the one or more of the sensors 112. The tremor signal is a representation of the tremulous activity generated in the brain and motor nerves that causes tremulous muscle activation leading to tremor in the hands, head, neck, legs, feet, and vocal cords.

In some embodiments, the sensor (e.g., IMU) 112 can include one or more of a gyroscope, accelerometer, and magnetometer. The sensor 112 can be affixed or integrated with the neuromodulation (e.g., neurostimulation) device 34. In an embodiment, the sensor 112 is an off the shelf component. In addition to its ordinary meaning, the sensor 112 can also include specific components as discussed below. For example, the sensor 112 can include one more sensors capable of collecting motion data. In an embodiment, the sensor 112 includes an accelerometer. In some embodiments, the sensor 112 can include multiple accelerometers to determine motion in multiple axes. Furthermore, the sensor 112 can also include one or more gyroscopes and/or magnetometer in additional embodiments. Since the sensor 112 can be integrated with the neurostimulation device 34, the sensor 112 can generate data from its sensors responsive to motion, movement, or vibration felt by the device 34. Furthermore, when the device 34 with the integrated sensor 112 is worn by a user, the sensor 112 can enable detection of voluntary and/or involuntary motion of the user.

The one or more sensors 112 may include an audio sensor, including but not limited to a microphone, audio transducer, or accelerometer, configured to measure biological processes, such as breathing, talking, or repetitive motion. Sensors, in some embodiments, sense parameters that are used to optimize neurostimulation and facilitate the introduction of variability in stimulation parameter(s) to reduce tolerance and/or habituation to the neurostimulation. As an example, EEG signals, brain activity and/or neuronal activity may be used in this manner. In one embodiment, variation in one or more parameters may be configured/introduced to generate a natural or desired characteristic of brain or neuronal activity over a time period for the treatment of movement, inflammatory, neurological and psychiatric disorders.

The device 34 can optionally include user interface components, such as a feedback generator 114 and a screen or display 46. The display 46 can provide instructions or information to users relating to calibration or therapy. The display 46 can also provide alerts, such an indication of response to therapy, for example. Alerts may also be provided using the feedback generator 114, which can provide haptic feedback to the user, such as upon initiation or termination of stimulation, for reminder alerts, to alert the user of a troubleshooting condition, to perform a tremor inducing activity to measure tremor motion, among others. Accordingly, the user interface components, such as the feedback generator 114 and the display 46 can provide audio, visual, and haptic feedback to the user. In certain embodiments, the feedback generator 114 and/or display 46 is configured for the user to provide satisfaction data to the device 34.

Furthermore, the device 34 can include communications hardware 118 for wireless or wired communication between the device 34 and an external system, such as the user interface device 150 discussed below. The communications hardware 118 can include an antenna as described above. The communications hardware 118 can also include an Ethernet or data bus interface for wired communications.

While the illustrated figure shows several components of the device 34, some of these components are optional and not required in all embodiments of the device 34. In some embodiments, a system can include a diagnostic device or component that does not include neuromodulation functionality. The diagnostic device could be a companion wearable device connected wirelessly through a connected cloud server, and include, for example, sensors such as cardiac activity, skin conductance, and/or motion sensors as described elsewhere herein.

In some embodiments, the device 34 can also be configured to deliver one, two or more of the following: magnetic, vibrational, mechanical, thermal, ultrasonic, or other forms of stimulation instead of, or in addition to electrical stimulation. Such stimulation can be delivered via one, two, or more effectors in contact with, or proximate the skin surface of the patient. However, in some embodiments, the device is configured to only deliver electrical stimulation, and is not configured to deliver one or more of magnetic, vibrational, mechanical, thermal, ultrasonic, or other forms of stimulation.

Figure 52B illustrates communications between the neurostimulation device 34 and a user interface device 150 over a communication link 130. The communication link 130 can be wired or wireless. The neuromodulation (e.g., neurostimulation) device 34 is capable of communicating and receiving instructions from the user interface device 150. The user interface device 150 can include a computing device. In some embodiments, the user interface device 150 is a mobile computing device, such as a mobile phone, a smartwatch, a tablet, or a wearable computer. The user interface device 150 can also include server computing systems that are remote from the neurostimulation device. In certain embodiments, the user interface device 150 can include a hardware processor(s) 152, a memory 154, a display 156, and power electronics 158. In some embodiments, the user interface device 150 can also include one or more sensors 160, such as sensors described elsewhere herein. Furthermore, in some instances, the user interface device 150 can generate an alert responsive to device issues or a response to therapy. The alert may be received from the neurostimulation device 34 via communication hardware 162.

In additional embodiments, data acquired from the one or more sensors 112 is processed by a combination of the hardware processor(s) 108 and hardware processor(s) 152. In further embodiments, data collected from one or more sensors 112 is transmitted to the user interface device 150 with little or no processing performed by the hardware processors 108. In some embodiments, the user interface device 150 can include a remote server that processes data and transmits signals back to the device 34 (e.g., via the cloud).

The device stimulation bursting frequency can be calibrated to a lateral postural hold task "wing-beating" or forward postural hold task for a predetermined time, e.g., 5-30 seconds (e.g., 20 seconds) for each subject. Other non-limiting examples of device parameters can be as disclosed elsewhere herein.

In some embodiments, stimulation may be applied to two or more nerves in an alternating manner at an interval defined by the tremor frequency (also referred to as burst frequency). In some embodiments, burst frequency is equal to the measured pathological tremor oscillation, which calculated from measured motion, muscle activity, or brain activity.

Various embodiments of the devices and/or systems discussed herein can stimulate nerves in an outer ear of a user, including but not limited to the auricular branch of the vagus nerve, great auricular nerve, auriculotemporal nerve, and/or lesser occipital nerve, among others. In one embodiment, a system can include a neuromodulation device on the wrist or other location of the arm to target a nerve of a subject (e.g., median nerve) and a neuromodulation device (such as any of the auricular devices described herein) in the ear to target the vagus nerve. In some implementations, each neuromodulation device in the system can communicate with each other via a wired or wireless connection. Multiple neuromodulation devices can provide synchronized stimulation to the multiple nerves. Stimulation may be, for example, burst, offset, or alternating between the multiple nerves. Modulation of the vagus nerve can be accomplished with the devices described herein, according to several embodiments. In some embodiments, the devices described herein are used to stimulate the autonomic system. In some embodiments, the devices described herein are used to balance the sympathetic/parasympathetic systems.

Variability of stimulation parameters, including but not limited to jitter or dither-like variability, can enhance the symptomatic and/or long-term reduction of tremor severity provided by the application of alternating stimulation between two or more peripheral nerves. This approach can overcome the challenge of variability observed in people with hand tremor between tremor episodes within an individual, or the variability observed between people in their brain response to peripheral nerve stimulation. Thus, several embodiments include systems and methods to reduce habituation and/or tolerance to stimulation by, for example, introducing variability in stimulation parameter(s).

Adding variation in burst frequency may account for natural variation in pathological tremor frequency. For example, in some cases pathological tremor frequency can change, for example, by more than 2 Hz between tasks and by up to 32% on the same task over time within an individual subject. Calibrating burst frequency to tremor frequency can improve therapeutic effect. However, as discussed above, it may be difficult to target particular tremor frequencies due to the natural variations. In some instances, it may not be suitable to continuously track the changing tremor characteristics using sensors discussed herein. It may consume too many computational resources and may also deplete battery. Therefore, the inventors realized that instead of focusing on a particular value or trying to exactly align to a pathological characteristic, adding variation in stimulation parameters, such as burst frequency, may enhance therapeutic benefit in treatment of conditions. Pathological characteristics can vary depending on the pathological condition. For example, for treatment of tremor, the characteristics of tremor may include tremor frequency, power, phase, amplitude, and the like. For example, for treatment of migraine, a 3 Hz burst frequency with a 150 Hz pulse frequency may override thalamocortical dysrhythmia in individuals. For example, for treatment of stroke, a 1 Hz burst frequency with a 10 Hz pulse frequency may reduce neuronal inhibition in the motor cortex that otherwise inhibits motor activity in individuals. In some instances, the characteristics may include physiological parameters, such as heart rate, respiration rate and/or content (respiratory rate; respiration phase; capnogram; oximetry; spirography), heart rate variability, blood pressure, and the like. The characteristics may also correspond to sympathetic and/or parasympathetic activity. Furthermore, the characteristics may correspond to neural oscillations. In some instances, neural oscillations may be observed in alpha, beta, delta, theta, gamma frequency bands. In some embodiments, EEG sensor is not required to probe these oscillations and provide therapeutic effect based on stimulation.

In some instances, variations will increase probability of alignment with the changing pathological characteristics during a portion of the therapy session, over time and across tasks. In some embodiments, one or more stimulation parameters are continuously varied over the course of the stimulation. Furthermore, in some instances, measuring tremor characteristics with one or more sensors is not required to provide a therapeutic effect. In addition to tremor, introduction of variability to treat conditions other than tremor are also provided (e.g., other movement disorders, migraine, stroke, other neurological disorders, etc.).

In additional embodiments, stimulation parameters are agnostic for any particular individual and may be varied within generally known therapeutic ranges during the course of stimulation. Adding variation in pulse frequency may account for individual differences in the brain response to peripheral nerve stimulation. For example, the evoked response generated in the ventral intermediate nucleus of the thalamus by median nerve stimulation was maximized at a pulse frequency of 50 Hz in some subjects and 100 Hz in other subjects. By varying pulse frequency throughout these range of values, the brain response is maximized during some portion of the therapy session for every individual, which may enhance therapeutic benefit. Varying pulse frequency during deep brain stimulation (DBS) therapy improved motor score outcomes, gait speed, and freezing of gait episodes in Parkinson's disease patients, compared to fixed frequency DBS. Finally, varying pulse frequency may produce natural stimulation-evoked sensations.

Adding variation in pulse intensity, current amplitude, voltage amplitude, or pulse width would be expected to change the extent of neuronal recruitment within the targeted nerves, with higher intensities and amplitudes, or longer pulse widths, increasing the extent of recruitment. These variations in nerve recruitment may vary the degree of activation in downstream neuronal sub-populations within the brain, which in turn could enhance therapeutic benefit, potentially by reducing the likelihood of neuronal adaptation or habituation to stimulation. In addition, varying pulse intensity or pulse width may produce more natural stimulation-evoked sensations than fixed stimulation. Systems and methods to reduce habituation and/or tolerance to stimulation are provided in several embodiments by, for example, introducing variability in stimulation parameter(s), as described herein. Habituation and/or tolerance to neurostimulation that occur in the treatment of movement, inflammatory, neurological and psychiatric disorders are treated in several embodiments.

Adding on/off periods in the stimulation waveform may enhance the therapeutic effects by increasing the desired desynchronization effect in downstream neuronal sub-populations within the brain.

Additionally, not to be limited by theory, variability in any of the above parameters can enhance the desired neuronal desynchronization effect that enhances therapeutic benefit (e.g., a lower tremor or symptom severity after application of stimulation).

Variability can be applied to one or more of the following parameters for stimulating a nerve including but not limited to burst frequency or alternating frequency, pulse frequency, pulse width, pulse spacing, intensity, current amplitude, voltage amplitude, duration of stimulation, on/off periods, or amplitude envelope periods. Variability can be applied across multiple stimulation parameters for stimulating a nerve including but not limited to simultaneous variation, braided variation, timescale variation, and adaptive learning. In certain embodiments, adaptive learning is employed in combination with the listed variations as well as other variations to improve neurostimulation therapy outcomes.

In some embodiments, the neuromodulation, e.g., neurostimulation device 34 can apply transcutaneous stimulation to a patient with tremor that is a candidate for implantable deep brain stimulation or thalamotomy. Tremor features and other sensor measurements of tremor severity will be used to assess response over a prespecified usage period, which could be 1 month or 3 months, or 5, 7, 14, 30, 60, or 90 days or more or less. Response to transcutaneous stimulation as assessed, for example, by algorithms described herein using sensor measurements from the device can advantageously provide input to a predictive model that provides an assessment of the patient's likelihood to respond to implantable deep brain stimulation or other implantable or non-implantable therapies.

In some embodiments, the neuromodulation, e.g., neurostimulation device 34 or a secondary device with sensors can collect motion data, or data from other sensors, when a tremor inducing task is being performed. The patient can be directly instructed to perform the task, for example via the display on the device or audio. In some embodiments, features of tremor inducing tasks are stored on the device and used to automatically activate sensors to measure and store data to memory during relevant tremor tasks. The period of time for measuring and storing data can be, for example, 10, 20, 30, 60, 90, 120 seconds, or 1, 2, 3, 5, 10, 15, 20, 30 minutes, or 1, 2, 3, 4, 5, 6, 7, 8 hours or more or less, or ranges incorporating any two of the foregoing values. Based on a training set of data from a cohort of previous wearers with tremor or another condition, the feature extraction engine can detect features that are correlated with response to stimulation such that the patient or physician can be presented with a quantitative and/or qualitative likelihood of the patient responding or not responding to treatment. This data can be measured in some cases prior to prescribing the neuromodulation, e.g., neurostimulation or during a trial period.

In another embodiment, features can be correlated with the type of tremor measured, such as resting tremor (associated with Parkinson's Disease), postural tremor, action tremor, intention tremor, rhythmic tremor (e.g., a single dominant frequency) or mixed tremor (e.g., multiple frequencies). The type of tremor most likely detected can be presented to the patient or physician as a diagnosis or informative assessment prior to receiving stimulation or to assess appropriateness of prescribing a neuromodulation, e.g., stimulation treatment. In another embodiment, various stimulation modes may be applied based on the tremor type determined; different modes could include changes in stimulation parameters, such as frequency, pulse width, amplitude, burst frequency, duration of stimulation, or time of day stimulation is applied. In one embodiment for a smartphone, tablet, or other device 150, the task to induce tremor can be included in an app that asks the patient to take a self-photograph, which has the patient perform a task that has both posture and intention actions.

In some embodiments, the neuromodulation, e.g., neurostimulation device 34 or a secondary device with sensors can collect motion data, or data from other sensors, can measure data over a longer period of time, for example 1, 2, 3, 4, 5, 10, 20, 30 weeks, 1, 2, 3, 6, 9, 12 months, or 1, 2, 3, 5, 10 years or more or less, or ranges incorporating any two of the foregoing values, to determine features, or biomarkers, associated with the onset of tremor diseases, such as essential tremor, Parkinson's disease, dystonia, multiple sclerosis, etc. Biomarkers could include specific changes in one or more features of the data over time, or one or more features crossing a predetermined threshold. In some embodiments, features of tremor inducing tasks have been stored on the device and used to automatically activate sensors when those tremor inducing tasks are being performed, to measure and store data to memory during relevant times.

In some embodiments, the neuromodulation device 34 can include the ability to track a user's motion data for the purpose of gauging one, two, or more tremor frequencies of a patient. The patient could have a single tremor frequency, or in some cases multiple discrete tremor frequencies that manifest when performing different tasks. Once the tremor frequencies are observed, they can be used as one of many seminal input parameters to a customized neuromodulation therapy. The therapy can be delivered, e.g., transcutaneously, via one, two, or more nerves (e.g., the median and radial or ulnar nerves, and/or other nerves disclosed elsewhere herein) in order to reduce or improve a condition of the patient, including but not limited to their tremor burden. In some embodiments, the therapy modulates afferent nerves, but not efferent nerves. In some embodiments, the therapy preferentially modulates afferent nerves. In some embodiments, the therapy does not involve functional electrical stimulation. The tremor frequency can be used to calibrate the patient's neuromodulation therapy, being used as a calibration frequency in some embodiments to set one or more parameters of the neuromodulation therapy, e.g., a burst envelope period. In some embodiments, the calibration frequency can be between, for example, about 4 Hz and about 12 Hz, between about 3 Hz and about 6 Hz, or about 3 Hz, 4 Hz, 5 Hz, 6 Hz, 7 Hz, 8 Hz, 9 Hz, 10 Hz, 11 Hz, or 12 Hz, or ranges including any two of the foregoing values. In some embodiments, the calibration process to match the tremor frequency (or otherwise be personalized or tailored to the user) includes one or more sessions. For example, in some embodiments, the calibration process comprises 1-5 sessions lasting 10-120 seconds e.g., three 20 second sessions. Post calibration, the therapy (e.g., neurostimulation) is tailored or personalized to the user. Specific examples for controlling the neurostimulation device 34 are described in more detail below.

Figure 53 illustrates a block diagram of another embodiment of a system 30 that provides peripheral nerve stimulation. In certain embodiments, the device and system senses a biological measure, a kinematic measure, and/or user satisfaction data. In certain embodiments, the system 30 uses the biological measure, the kinematic measure, and/or the user satisfaction data to customize or modify the delivery of an electrical stimulus.

In some embodiments, the system 30 comprises a pulse generator 201. In certain embodiments, the pulse generator 201 delivers electrical stimulation to a nerve through one or more skin interfaces 203. In certain embodiments, the one or more skin interfaces 203 can be an electrode 74 as described elsewhere herein. In certain embodiments, the one or more skin interfaces 203 sit adjacent to one or more target peripheral nerves. A controller 200 receive one on more signals generated by one or more sensors 112 to control timing and parameters of stimulation. In certain embodiments, the controller 200 uses instructions stored in the memory 209 to coordinate receiving signals from the one or more sensors 112. In certain embodiments, the controller 200 uses the received signal to control stimulation delivered by the pulse generator 201. The memory 209 in the system 216 can store signal data from the sensors 112.

In certain embodiments, the system 30 has a communication module 210 to transmit data to other devices or a remote server via standard wired or wireless communication protocols. In certain embodiments, the system 30 is powered by a battery 214. In certain embodiments, the system 30 has a user interface 46. In certain embodiments, the user interface 46 allows the user to receive feedback from the system 30. In certain embodiments, the user interface 46 allows the user to provide input to the system 30 via, e.g., one or more buttons. In certain embodiments, the user provides satisfaction data via the user interface 46. For example, the user can provide input to the user interface 46 in the form of a patient session impression of improvement (PSII) score and/or a patient satisfaction scope. In certain embodiments, the user interface 46 allows a user to receive instructions, feedback, and control aspects of the delivered stimulation, such as intensity of the stimulation. In certain embodiments, the user manually enters the patient session impression of improvement (PSII) score to indicate one or more of 1) Improved, 2) No Change, or 3) Worse patient satisfaction with the therapy or calibration session. This information is then used, in one embodiment, to adjust therapy (e.g., neurostimulation) parameters.

In certain embodiments, the controller 200 can receive kinematic and/or satisfaction data to determine a method for varying multiple stimulation parameters based on adaptive learning as disclosed herein. In certain embodiments, the controller 200 causes the device 34 to adjust one or more parameters of a first electrical stimulus based at least in part on the kinematic and/or satisfaction data.

In some embodiments, the controller 200 of the wearable system 30 employs a treatment algorithm. For example, in certain embodiments, the user selects from a plurality of different types of therapy waveforms. The controller 200 can employ predictive capability to determine a best of the plurality of waveforms (e.g., transcutaneous afferent patterned stimulation (e.g., CALA TAPS^{™}), burst frequency jitter (BFV), and pulse frequency jitter (PFV)) for the specific patient. For example, in some embodiments, the controller 200 employs machine learning to predict improvement in efficacy and/or patient satisfaction ratings for two, three or more waveforms. The controller 200 can use the predicted improvements in efficacy and/or patient satisfaction ratings to recommend an improved therapy. The improved therapy can include identify the best or desired waveform for the specific patient. The improved waveform can be retrieved from the base station during charging of the neurostimulation device, in some embodiments.

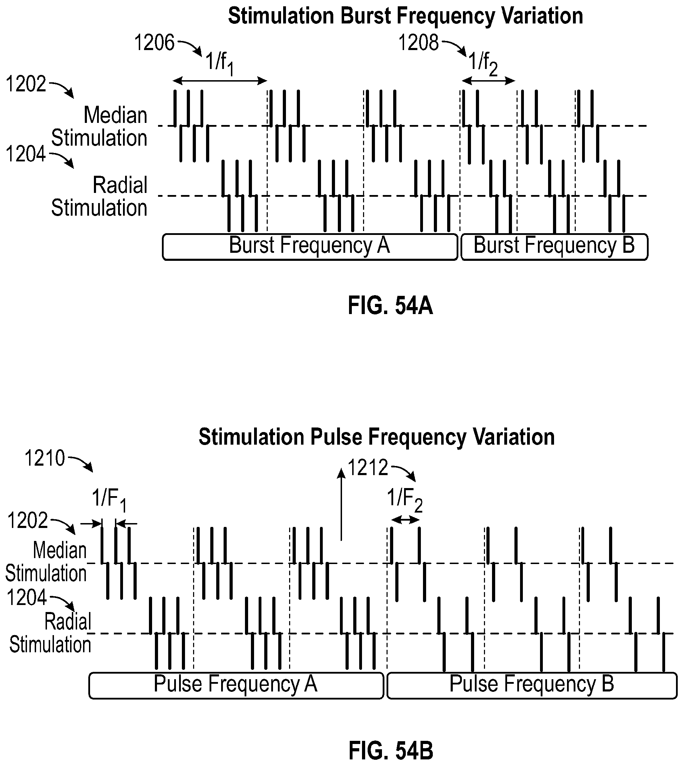

Figures 54A-C2 illustrate examples of how stimulation parameters (e.g., burst frequency, pulse frequency, and pulse phase) are varied between two or more prespecified values as stimulation is alternated across two nerves (e.g., median and radial nerve). The plots show patterns of current delivered by the device 34 over time.

Figure 54A illustrates an embodiment of the device 34 that delivers patterned stimulation to the median nerve 1202 and radial nerve 1204 where burst frequency is varied after a prespecified time period or prespecified number of bursts. As is illustrated in Figure 54A, the burst frequency is initially burst frequency A with a period of 1/f1 1206. The burst frequency subsequently changes to burst frequency B with a different period of 1/f2 1208. Figure 54A is only exemplary and is not intended to limit the variations in burst frequency to the illustrated values or the number of different burst frequencies. Further, while Figure 54A illustrates the variation occurring across multiple nerves (e.g., median and radial nerves), the disclosure is not so limited. The disclosed variations can be applied to only a single nerve.

In some embodiments, burst frequency variability is centered on an about, at least about, or no more than about 0.1, 0.2, 0.25, 0.3, 0.4, 0.5, 1, 1.5, 2, 2.5, 3, 3.5, 4, 4.5, 5, 5.5, or 6 Hz or more or less window (or ranges including any two of the foregoing values), or any combination thereof, around a calibration frequency measured from a tremor-inducing task, such as a postural hold. In certain embodiments, if the measured tremor frequency is at a lower edge of a partial tremor frequency range (e.g., a 3-12 Hz window), the burst frequency variation window would not go below 3 Hz. In certain embodiments, if the measured tremor frequency is at the higher edge of a partial tremor frequency range (e.g., a 3-12 Hz window), the burst frequency variation window would not go above 12 Hz. In an alternative embodiment, burst frequency variability is applied within the full or partial tremor frequency range, for example between 3-12 Hz for essential tremor. This alternative embodiment may have the advantage of not requiring the user to perform a tremor inducing task for calibration. In yet another embodiment, the range of values for burst frequency variability is set based on the minimum and maximum tremor frequencies measured from multiple tremor-inducing task measurements. In some embodiments, burst frequency variability can avoid exact alignment to the pathological oscillation frequency over time and enhance the therapeutic response compared to a constant burst frequency. In some embodiments, the rate of change of the burst frequency parameter may be between 0.001 Hz/s (i.e., slowest rate of change of burst frequency being in increments of 0.1 Hz every 100 sec) to 100 Hz/s (i.e., fastest rate of change of burst frequency being in increments of 8 Hz burst frequency change every tremor cycle and rounding up).

Figure 54B illustrates an embodiment of the device 34 that delivers patterned stimulation to the median nerve 1202 and radial nerve 1204 where pulse frequency is varied after a prespecified time period or prespecified number of bursts. As is illustrated in Figure 54B, the pulse frequency is initially pulse frequency A with a period of 1/F1 1210. The pulse frequency subsequently changes to pulse frequency B with a different period of 1/F2 1212. Figure 54B is only exemplary and is not intended to limit the variations in pulse frequency to the illustrated values or number of pulse frequencies. Further, while Figure 54B illustrates the variation occurring across multiple nerves (e.g., median and radial nerves), the disclosure is not so limited. The disclosed variations can be applied to only a single nerve.

In some embodiments, the pulse frequency of electrical stimulation applied to a peripheral nerve or neuron can govern how frequently the stimulated nerve or neuron generates an action potential. In some cases, peripheral nerve fibers can be activated to generate an action potential with every stimulation pulse at pulse frequencies of less than approximately 1,000 Hz, if the stimulation pulse width and amplitude are sufficiently high. In some cases, stimulation of the median nerve with pulse frequencies of 5, 50, 100, 150, and 200 Hz can evoke a response of the VIM thalamus, as measured with implanted microelectrodes during a surgical procedure. Moreover, the pulse frequency that generates the maximal amplitude evoked response of the VIM thalamus can vary across subjects. In some embodiments, pulse frequency is varied between 5-200, 5-150, 5-100, 5-50, 50-200, 50-150, 50-100, 100-200, 100-150, or 150-200 Hz (or ranges including any two of the foregoing values), which can enhance therapeutic response compared to a constant pulse frequency. Changes in pulse frequency may be implemented by changing the timing of pulse delivery directly, or by keeping the timing fixed and alternating stimulation amplitude on a pulse-to-pulse basis to change the effective pulse frequency. For example, setting every 1 of 2 pulses to a low stimulation amplitude, which is subthreshold for recruitment of neurons or nerves, can reduce the effective pulse frequency by ½. In some embodiments, the rate of change of the pulse frequency parameter may be between 0.001-10,000 Hz/s. In some embodiments, varying pulse frequency may generate activity in the brain that modulates pathological cortical dynamics associated with hand tremor. An additional advantage of varying pulse frequency is that this type of stimulation can elicit a more natural paresthesia sensations, similar to tapping, pressure, touch, and/or vibration sensations experienced during daily life.

In one embodiment the pulse frequency may be from about 1 to about 5000 Hz, about 1 Hz to about 500 Hz, about 5 Hz to about 50 Hz, about 50 Hz to about 300 Hz, or about 150 Hz, or other ranges including any two of the foregoing values. In some embodiments, the pulse frequency may be from 1 kHz to 20 kHz.

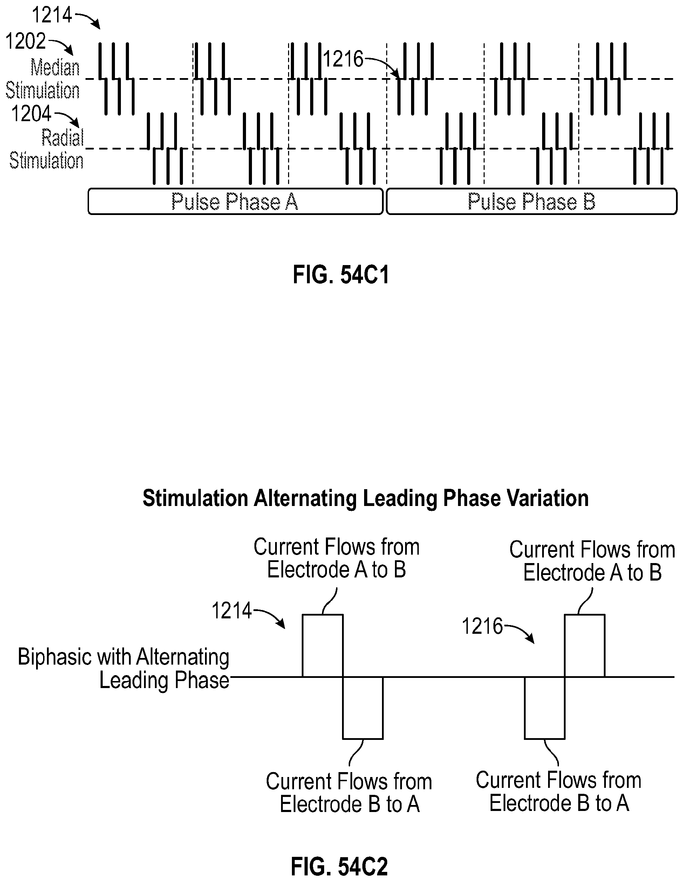

Figures 54C1-C2 illustrate embodiments of the device 34 that deliver biphasic patterned stimulation to the median nerve 1202 and radial nerve 1204 where the leading pulse phase changes or alternates (e.g., one or more pulses or bursts of a cathodic-first phase of current flowing from electrode 1 to 2 followed by one or more pulses or bursts of an anodic-first phase of current flowing from electrode 2 to 1 or vice versa) after (1) a prespecified time period (Figure 54C1), (b) a prespecified number of bursts (Figure 54C1), or (c) a prespecified number of pulses (Figure 54C2). In this way, at least some of the stimulation pulses delivered will have a different leading first phase as opposed to all of the stimulation pulses having a constant cathodic-first phase pattern or a constant anodic first phase pattern (e.g., Figure 54A).

As is illustrated in Figure 54C1, the leading pulse phase is pulse phase A 1214 (e.g., a current flows initially during the pulse from electrode 1 to electrode 2) during each pulse for a prespecified time period or number of bursts. In the illustrated embodiment of Figure 54C1, the leading pulse phase A 1214 is maintained for a series of three bursts with each burst comprising three pulses. The leading pulse phase subsequently alternates to pulse phase B 1216 (e.g., a current flows initially during the pulse from electrode 2 to electrode 1).

In the illustrated embodiment of Figure 54C1, the leading pulse phase B 1216 is maintained for a series of three bursts with each burst comprising three pulses. Figure 54C1 is only exemplary and is not intended to limit the variations in leading pulse phase to the illustrated numbers of bursts or pulses. Further, while Figure 54C1 illustrates the variation occurring across multiple nerves (e.g., median and radial nerves), the disclosure is not so limited. The disclosed variations can be applied to only a single nerve.

As is illustrated in Figure 54C2, the leading pulse phase is pulse phase A 1214 (e.g., a current flows initially during the pulse from electrode 1 to electrode 2) for one pulse. The leading pulse phase subsequently alternates to pulse phase B 1216 (e.g., a current flows initially during the pulse from electrode 2 to electrode 1) during a second pulse. This alternating pattern can continually repeat at an interval. Figure 54C2 is only exemplary and is not intended to limit the variations in pulse phase to the illustrated number of pulses or interval for alternating between leading pulse phases. For example, the phase of the leading first pulse can be repeated for two or more pulses before alternating to leading pulse phase B 1216. Further, the leading phase of the second pulse can be repeated for two or more pulses before alternating back to the leading phase of the first pulse. Further, while Figure 54C2 illustrates the variation occurring across a single nerve, the disclosure is not so limited. The disclosed variations can be applied to multiple nerves (e.g., median and radial or ulnar nerves).

In some embodiments, prolonged percutaneous stimulation sessions employing a constant pattern of a leading cathodic or anodic first phase may cause electro-chemical changes in the electrode-skin interface even though each pulse is intended to be charge balanced by flowing current in one direction and then reversing the current flow during the pulse (e.g., biphasic). Thus, electro-chemical changes may occur during biphasic operation causing discomfort and adverse biological effects (e.g., skin irritations) due to the movement of charged molecules within the skin caused by the flow of current across the skin. Alternating the leading phase of at least some pulses within the stimulation session such as illustrated in Figures 54C1 and 54C2 can mitigate against such adverse biological effects.

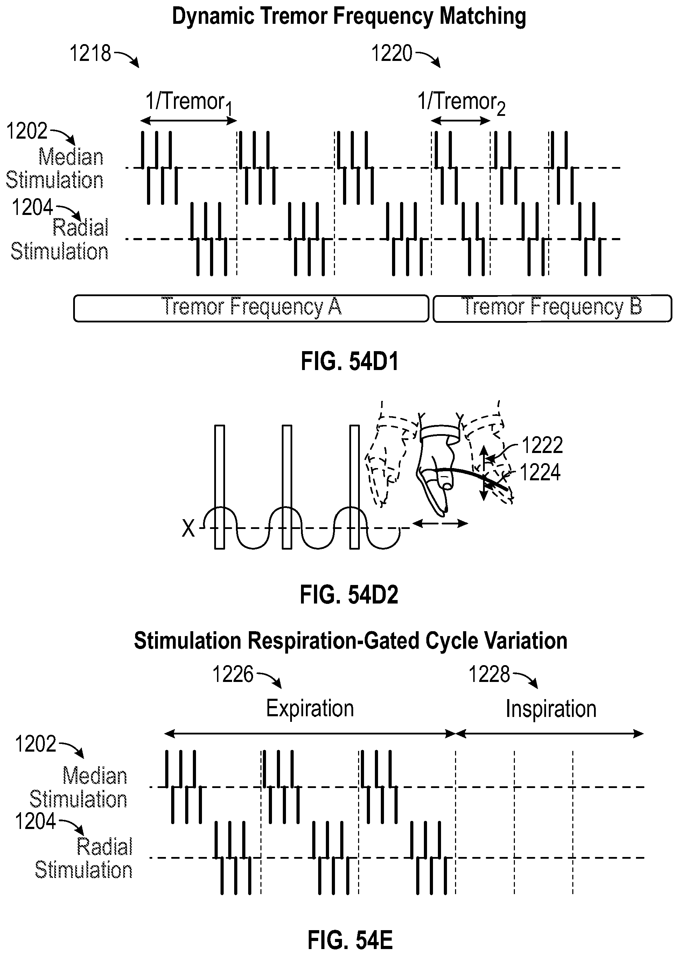

Figure 54D1 illustrates an embodiment of the device 34 that employs dynamic tremor frequency matching. In some embodiments, the device 34 dynamically varies the burst frequency of the patterned stimulation to the median nerve 1202 and/or radial nerve 1204 based at least in part on changes in tremor frequency. In some embodiments, the frequency of the stimulation to the median nerve 1202 and the radial nerve 1204 dynamically tracks real-time, measured changes in the tremor frequency. As is illustrated in Figure 54D2, in some embodiments, the frequency of the stimulation to a first nerve (e.g., the median nerve 1202) tracks a first phase of the tremor (e.g., hand moving in downward direction 1224) while the stimulation to a second nerve (e.g., the radial nerve 1204) tracks a different phase of the tremor (e.g., hand moving in upward direction 1222).

In some embodiments, the tremor frequency can be between, for example, about 4 Hz and about 12 Hz, between about 3 Hz and about 6 Hz, or about 3 Hz, 4 Hz, 5 Hz, 6 Hz, 7 Hz, 8 Hz, 9 Hz, 10 Hz, 11 Hz, or 12 Hz, or ranges including any two of the foregoing values. In some cases, pathological tremor frequency can change, for example, by more than 2 Hz between tasks and by up to 32% on the same task over time for an individual patient. In some embodiments, phase-locking burst frequency to tremor frequency can improve therapeutic effect.

As is illustrated in Figure 54D1, the burst frequency for the median and radial stimulation can initially match tremor frequency A with a period of 1/tremor 1218. The tremor frequency can then change to tremor frequency B. The burst frequency can change to tremor frequency B with a different period of 1/tremor 1220 so as to continue matching the frequency of the tremor.

As is illustrated in Figure 54D2, in some embodiments, the timing of median and radial nerve stimulation can be determined based on the measured, real-time phases of the patient's tremor. For example, median nerve stimulation could be delivered while the tremor is between phases 0-180 degrees (e.g., hand moving in downward direction 324), while radial nerve stimulation could be delivered when the tremor is between phases 180-360 degrees (e.g., hand moving in upward direction 1222). In other embodiments, the radial nerve stimulation can be delivered while the tremor is between phases 0-180 degrees (e.g., hand moving in downward direction 1224), while medial nerve stimulation could be delivered when the tremor is between phases 180-360 degrees (e.g., hand moving in upward direction 1222).

In some embodiments, the durations of the different phases are asymmetrical. For example, in some embodiments, the duration of the first phase (e.g., hand moving in downward direction 1224) is not the same as the duration of the second phase (e.g., hand moving in upward direction 1222). In some embodiments, the device 34 delivers asymmetric stimulation to the first and second nerves based at least in part on the asymmetric phases of the tremor. Figures 54D1 and 54D2 are only exemplary and are not intended to limit the variations in the associated timing between nerve stimulation and the real-time phase of the patient's tremor. Further, while Figures 54D1 and 54D2 illustrate the variation occurring across multiple nerves (e.g., median and radial nerves), the disclosure is not so limited. The disclosed variations can be applied to only a single nerve.

In some embodiments, the one or more sensors 112 of the device 34 tracks the patient's motion data for the purpose of gauging, real-time, a tremor frequency of the patient and/or phases of the tremor. Once the tremor frequency is observed, the device 34 can use the frequency as a seminal input parameter. The one or more sensors 112 (e.g., inertial measurement unit (IMU), accelerometer, gyroscope, etc.) can measure motion of the patient's extremity for the device 34 to generate motion data; determining tremor frequency from the motion data; and setting the burst frequency to match or closely match (e.g., phase-locked) the measured motion. For example, in some embodiments, an accelerometer configured as the sensor 112 passively measures tremor during a treatment session. In some embodiments, the device 34 continuously tracks the changing tremor characteristics using the one or more sensors 112. In some embodiments, the one or more hardware processor(s) 108, 152 analyze the phase and trigger median 1202 or radial 1204 nerve stimulation accordingly.

In some embodiments, as illustrated in Figure 54D2, the phase cutoffs (e.g., 0 and 180 degrees) for switching between median 1202 and radial 1204 nerve stimulation can be personalized for the patient. For example, a plurality of different phase cutoffs and ranges could be employed (e.g., 0, 30, 60, 90, 120, 150, 180, 210, 240, 270, 300, 330, and 360 degrees or ranges including any two of the foregoing values) for a series of stimulation sessions. The device 34 could employ the phase cutoffs which produce the best tremor relief. As mentioned above, the phase cutoff can be symmetric or asymmetric depending on, for example, the measured phases of the tremor.

In some embodiments, the dynamic tremor frequency matching burst frequency is centered on about, at least about, or no more than about 0.1, 0.2, 0.25, 0.3, 0.4, 0.5, 1, 1.5, 2, 2.5, 3, 3.5, 4, 4.5, 5, 5.5, or 6 Hz or more or less window (or ranges including any two of the foregoing values), or any combination thereof, around a measured tremor frequency. In certain embodiments, burst frequency variability dynamically matches the pathological oscillation frequency over time. In some embodiments, the rate of change of the measured tremor frequency may be between 0.001 Hz/s (i.e., slowest rate of change of burst frequency being in increments of 0.1 Hz every 100 sec) to 100 Hz/s (i.e., fastest rate of change of burst frequency being in increments of 8 Hz burst frequency change every tremor cycle and rounding up).

Figure 54E illustrates an embodiment of the device 34 that delivers patterned stimulation to a first nerve (e.g., the median nerve 1202) and a second nerve (e.g., the radial nerve 1204) based at least in part on the patient's respiratory cycle (e.g., respiratory gating). As is illustrated in Figure 54E, in some embodiments, the timing of median and radial nerve stimulation can be determined based on the measured, real-time phases of the respiratory cycle. Delivering stimulation during a first portion of the respiration cycle and then discontinuing the stimulation during a second portion of the respiration cycle (or vice versa) may enhance autonomic modulatory effects. In some embodiments, a first target nerve can be modulated during an inspiratory phase of the respiratory cycle, and then no stimulation is applied to the first target nerve during an expiratory phase of the respiratory cycle. In this way, the device 34 can be configured to synchronize/gate the stimulation to one or more particular phases of the respiratory cycle.

In some embodiments, stimulation to the median nerve 1202 and the radial nerve 1204 tracks measured changes in the respiratory cycle. As is illustrated in Figure 54E, in some embodiments, the stimulation to the first and second nerves (e.g., the median nerve 1202 and the radial nerve 1204) occurs during a first portion of the respiration cycle (e.g., expiration 1226) and is discontinued during a second portion of the respiration cycle (e.g., inspiration 1228). In some embodiments, the cycle can then repeat by restimulating the first and second nerves during the next expiration and discontinuing the stimulation during the following inspiration. While the first portion (on stimulation) and second portion (off stimulation) are illustrated as corresponding to the expiration and inspiration phases of the respiratory cycle, the disclosure is not so limited. The first portion and the second portion can correspond to any parts of the respiratory cycle. In some embodiments, the device 34 can analyze a voltage or other signal from the sensor 112 in real-time and can detect different features of the respiratory cycle of the patient. The features detected by the sensor 112 can include, for example, peaks, troughs, and slopes reaching, exceeding, or being less than a predetermined value, for example. Further, in some embodiments, the respiratory cycle can be split into three or more parts (e.g., peaks, troughs, slopes, etc.) based on data received by the sensor 112 with each part corresponding to on or off stimulation.

In some embodiments, the sensor 112 is carried by a respiratory detection device (e.g., a respiration belt) worn by the patient. In some embodiments, the respiratory detection device can further include a communication module, which may be cellular, Bluetooth etc., to communicate with the device 34. In some embodiments, the timing of the stimulation can depend on the algorithm used to trigger the stimulation off of one or more of the measured biological signals (e.g., respiratory cycle) received from the sensor 112. For example, in certain embodiments, the stimulation is triggered based at least in part on whether the burst is a fixed duration, is a percentage of one or more measured biological signals (e.g., respiratory cycle), terminates at a detected phase of a cyclical biological signal (e.g., respiratory cycle), or is based on some other algorithm implemented in the device 34. For example, in some embodiments, the device 34 triggers stimulation when the inspiration and/or expiration phase of the respiratory cycle is detected and continues for at least about, about, or no more than about 5%, 10%, 15%, 20%, 25%, 30%, 35%, 40%, 45%, 50%, 55%, 60%, 65%, 70%, 75%, 80%, 85%, 90%, or 95% of the average measured respiratory interval or ranges including any two of the aforementioned values.

In some embodiments, the device 34 can identify specific points on the respiratory signal that may be more receptive to stimulation. In some embodiments, the stimulation is synchronized to respiratory activity, but the stimulation is not necessarily configured to affect or substantially affect respiratory function (e.g., one or more or respiratory rate, tidal volume, or minute ventilation). For example, dual peripheral nerve stimulation (e.g., medial 1202 and radial 1204 nerve stimulation) can be synchronized to two different phases of the respiratory cycle, to treat the patient without affecting or substantially affecting the patient's respiratory function, and/or affecting or substantially affecting the patient's heart rate or rhythm in some embodiments.

In some embodiments, the stimulation can be synchronized to early expiration, late expiration, early inspiration, and/or late inspiration. The stimulation could also be synchronized to, for example, the 1st, 2nd, 3rd, 4th, 5th, 6th, 7th, 8th, 9th, and/or 10th decile chronologically of an inspiratory and/or expiratory cycle, including ranges and/or combinations of any of the foregoing values. In some embodiments the stimulation could be synchronized continuously to the targeted phase(s) of each respiratory cycle, every other respiratory cycle, every third respiratory cycle, on for a predetermined or calculated number of targeted phases(s) of respiratory cycles and off for the same or different predetermined or calculated number of targeted phases(s) of respiratory cycles, or other patterns depending on the desired clinical result. In some embodiments, the stimulation could include a first stimulation mode during a first portion of the respiratory cycle, e.g., expiration, and a second, different stimulation mode during a second portion of the respiratory cycle, e.g., another part of expiration and/or inspiration.

In some embodiments, the durations of the different phases of the respiratory cycle are asymmetrical. For example, in some embodiments, the duration of the first phase (e.g., expiration 1226) is not the same as the duration of the second phase (e.g., inspiration 1228). In some embodiments, the device 34 delivers asymmetric stimulation to the first and second nerves based at least in part on the asymmetric phases of the respiratory cycle. Figure 54E is only exemplary and is not intended to limit the variations in the associated timing between nerve stimulation and the real-time phase of the patient's respiratory cycle. Further, while Figure 54E illustrates the variation occurring across multiple nerves (e.g., median and radial nerves), the disclosure is not so limited. The disclosed variations can be applied to only a single nerve.

In some embodiments, the one or more sensors 112 of the device 34 track the patient's motion data for the purpose of gauging, real-time, phases of a respiratory cycle of the patient. Once the respiratory cycle is observed, the device 34 can use the frequency as a seminal input parameter. The one or more sensors 112 can measure respiratory rate and/or content (respiration rate; respiration phase; capnogram; oximetry; spirography), of the patient for the device 34 to generate respiratory data; determining phases of the respiratory cycle from the respiratory data; and turning the stimulation on or off based at least in part on the measured respiration. For example, in some embodiments, the respiratory detection device passively measures respiration during a treatment session. In some embodiments, the device 34 continuously tracks the changing respiration characteristics using the respiratory detection device. In some embodiments, the one or more hardware processor(s) 108, 152 analyze the respiratory cycle and trigger median 1202 or radial 1204 nerve stimulation accordingly.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under", or "beneath" other elements or features would then be oriented "over" the other elements or features. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps. However, some embodiments can consist or consist essentially of any number of stated elements or steps disclosed herein.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure.

## Claims

1. A wearable system (30) having a removable neurostimulation device to enhance mechanical securement, the removable neurostimulation device being configured to modulate one or more peripheral nerves of a user, the wearable system comprising:
(i) a band (32) having an outside and an inside, the outside being viewable by the user and the inside facing skin of the user when the band is worn by the user, the band comprising;
an electrode system (42) supported by the band and having an inner side and an outer side, the inner side comprising at least one electrode (74) for each of the one or more peripheral nerves to be modulated;
a frame (44) mechanically and electrically coupled to the band, the frame further comprising an engagement structure (40, 52, 92) and one or more electrical contacts (90); and one or more electrical traces (94) extending between the frame and the at least one electrode;
and(ii) a removable neurostimulation device (34) having an upper surface, a lower surface, and an outer wall disposed therebetween, the outer wall comprising one or more electrical contacts (86), the outer wall being sized and shaped to be mechanically secured within the engagement structure of the frame;
wherein the one or more electrical contacts of the removable neurostimulation device are configured to electrically interface with the one or more electrical contacts of the frame when the outer wall is mechanically secured within the engagement structure of the frame; and
when the removable neurostimulation device is inserted into the frame from the inside of the band the engagement structure prevents the removable neurostimulation device from passing entirely through the frame,
wherein the removable neurostimulation device is configured to modulate one or more peripheral nerves of a user through delivery of electrical signals.

2. The wearable system of Claim 1, wherein the engagement structure is an abutment surface (52), wherein the removable neurostimulation device comprises a contact surface shaped and sized to contact the abutment surface when the removable neurostimulation device is secured to the band, and wherein the band is replaceable with an alternate band.

3. The wearable system of Claim 1, wherein the engagement structure is an opening (50).

4. The wearable system of Claim 3, wherein the removable neurostimulation device further comprises a screen (46), the screen being viewable inside the opening from outside the band when the removable neurostimulation device is inserted into the frame.

5. The wearable system of Claim 3, wherein the outer wall has a shape of a step, the step comprising a riser and tread, and wherein a circumference of the riser is less than an inner circumference of the opening.

6. The wearable system of Claim 5, wherein the tread prevents the removable neurostimulation device from passing entirely through the frame when the removable neurostimulation device is captured by the band.

7. The wearable system of any one of Claims 1 to 6, wherein the at least one electrode comprises a return or ground electrode configured to be electrically coupled to the user.

8. The wearable system of any one of Claims 1 to 6, wherein the at least one electrode comprises at least a first electrode and a second electrode, the first electrode being configured to stimulate the median nerve of the user and the second electrode being configured to stimulate the radial or ulnar nerve of the user.

9. The wearable system of any one of Claims 1 to 6, wherein at least a portion of the outer wall is curved between the upper and lower surfaces.

10. The wearable system of any one of Claims 1 to 6, wherein at least a portion of the outer wall is flat between the upper and lower surfaces.

11. The wearable system of any one of Claims 1 to 6, wherein the band is configured to be tightened about a limb forcing the at least one electrode firmly against the skin of the user.

12. The wearable system of any one of Claims 1 to 6, wherein the electrical signals delivered to the one or more peripheral nerves of the user varies a burst frequency after a prespecified time period.

13. The wearable system of any one of Claims 1 to 6, wherein the electrical signals delivered to the one or more peripheral nerves of the user varies a burst frequency after a prespecified number of bursts.

14. The wearable system of any one of Claims 1 to 6, wherein the electrical signals delivered to the one or more peripheral nerves of the user varies a pulse frequency after a prespecified time period.

15. The wearable system of any one of Claims 1 to 6, wherein the electrical signals delivered to the one or more peripheral nerves of the user varies a pulse frequency after a prespecified number of bursts.

## Patentansprüche

1. Tragbares System (30) mit einer abnehmbaren Neurostimulationsvorrichtung zur Verbesserung der mechanischen Befestigung, wobei die abnehmbare Neurostimulationsvorrichtung dafür dafür konfiguriert ist, einen oder mehrere periphere Nerven eines Benutzers zu stimulieren, wobei das tragbare System umfasst:
(i) ein Band (32) mit einer Außenseite und einer Innenseite, wobei die Außenseite für den Benutzer sichtbar ist und die Innenseite der Haut des Benutzers zugewandt ist, wenn das Band vom Benutzer getragen wird, wobei das Band umfasst:
ein vom Band getragenes Elektrodensystem (42) mit einer Innenseite und einer Außenseite, wobei die Innenseite mindestens eine Elektrode (74) für jeden der einen oder mehreren zu stimulierenden peripheren Nerven umfasst;
einen Rahmen (44), der mechanisch und elektrisch mit dem Band gekoppelt ist, wobei der Rahmen ferner eine Eingriffsstruktur (40, 52, 92) und einen oder mehrere elektrische Kontakte (90) umfasst; sowie eine oder mehrere elektrische Leiterbahnen (94), die sich zwischen dem Rahmen und der mindestens einen Elektrode erstrecken;
und (ii) eine entfernbare Neurostimulationsvorrichtung (34) mit einer Oberseite, einer Unterseite und einer dazwischen angeordneten Außenwand, wobei die Außenwand einen oder mehrere elektrische Kontakte (86) umfasst, wobei die Außenwand so dimensioniert und geformt ist, dass sie mechanisch in der Eingriffsstruktur des Rahmens befestigt werden kann;
wobei der eine oder die mehreren elektrischen Kontakte der abnehmbaren Neurostimulationsvorrichtung so konfiguriert sind, dass sie elektrisch mit dem einen oder den mehreren elektrischen Kontakten des Rahmens verbunden werden, wenn die Außenwand mechanisch in der Eingriffsstruktur des Rahmens befestigt ist; und
wenn die abnehmbare Neurostimulationsvorrichtung von der Innenseite des Bandes her in den Rahmen eingeführt wird, verhindert die Verriegelungsstruktur, dass die abnehmbare Neurostimulationsvorrichtung den Rahmen vollständig durchläuft,
wobei die abnehmbare Neurostimulationsvorrichtung so konfiguriert ist, dass sie einen oder mehrere periphere Nerven eines Benutzers durch die Abgabe elektrischer Signale stimuliert.

2. Tragbares System nach Anspruch 1, wobei die Eingriffsstruktur eine Anlagefläche (52) ist, wobei die abnehmbare Neurostimulationsvorrichtung eine Kontaktfläche umfasst, die so geformt und dimensioniert ist, dass sie die Anlagefläche berührt, wenn die abnehmbare Neurostimulationsvorrichtung am Band befestigt ist, und wobei das Band durch ein alternatives Band austauschbar ist.

3. Tragbares System nach Anspruch 1, wobei die Eingriffsstruktur eine Öffnung (50) ist.

4. Tragbares System nach Anspruch 3, wobei die abnehmbare Neurostimulationsvorrichtung ferner einen Bildschirm (46) umfasst, wobei der Bildschirm von außerhalb des Bandes in die Öffnung hinein sichtbar ist, wenn die abnehmbare Neurostimulationsvorrichtung in den Rahmen eingesetzt ist.

5. Tragbares System nach Anspruch 3, wobei die Außenwand die Form einer Stufe aufweist, wobei die Stufe eine Setzstufe und eine Trittfläche umfasst, und wobei der Umfang der Setzstufe kleiner ist als der Innenumfang der Öffnung.

6. Tragbares System nach Anspruch 5, wobei die Trittfläche verhindert, dass die abnehmbare Neurostimulationsvorrichtung vollständig durch den Rahmen hindurchgleitet, wenn die abnehmbare Neurostimulationsvorrichtung vom Band erfasst wird.

7. Tragbares System nach einem der Ansprüche 1 bis 6, wobei die mindestens eine Elektrode eine Rückleitungs- oder Erdungselektrode umfasst, die so konfiguriert ist, dass sie elektrisch mit dem Benutzer gekoppelt ist.

8. Tragbares System nach einem der Ansprüche 1 bis 6, wobei die mindestens eine Elektrode mindestens eine erste Elektrode und eine zweite Elektrode umfasst, wobei die erste Elektrode so konfiguriert ist, dass sie den Nervus medianus des Benutzers stimuliert, und die zweite Elektrode so konfiguriert ist, dass sie den Nervus radialis oder den Nervus ulnaris des Benutzers stimuliert.

9. Tragbares System nach einem der Ansprüche 1 bis 6, wobei mindestens ein Abschnitt der Außenwand zwischen der Ober- und der Unterseite gekrümmt ist.

10. Tragbares System nach einem der Ansprüche 1 bis 6, wobei mindestens ein Abschnitt der Außenwand zwischen der Ober- und der Unterseite flach ist.

11. Tragbares System nach einem der Ansprüche 1 bis 6, wobei das Band so konfiguriert ist, dass es um eine Extremität festgezogen wird, wodurch die mindestens eine Elektrode fest gegen die Haut des Benutzers gedrückt wird.

12. Tragbares System nach einem der Ansprüche 1 bis 6, wobei die an den einen oder die mehreren peripheren Nerven des Benutzers abgegebenen elektrischen Signale nach einem vorbestimmten Zeitraum eine Burstfrequenz variieren.

13. Tragbares System nach einem der Ansprüche 1 bis 6, wobei die an den einen oder die mehreren peripheren Nerven des Benutzers abgegebenen elektrischen Signale nach einer vorbestimmten Anzahl von Bursts eine Burstfrequenz variieren.

14. Tragbares System nach einem der Ansprüche 1 bis 6, wobei die an den einen oder die mehreren peripheren Nerven des Benutzers abgegebenen elektrischen Signale nach einem vorbestimmten Zeitraum eine Impulsfrequenz variieren.

15. Tragbares System nach einem der Ansprüche 1 bis 6, wobei die an den einen oder die mehreren peripheren Nerven des Benutzers abgegebenen elektrischen Signale nach einer vorbestimmten Anzahl von Bursts eine Impulsfrequenz variieren.

## Revendications

1. Système à porter sur soi (30) comprenant un dispositif de neurostimulation amovible pour améliorer la fixation mécanique, le dispositif de neurostimulation amovible étant conçu pour moduler un ou plusieurs nerfs périphériques d'un utilisateur, le système à porter sur soi comprenant :
(i) un bracelet (32) présentant une face extérieure et une face intérieure, la face extérieure étant visible de l'utilisateur et la face intérieure étant en contact avec la peau de l'utilisateur lorsque celui-ci porte le bracelet, le bracelet comprenant :
un système d'électrodes (42) supporté par le bracelet et présentant un côté intérieur et un côté extérieur, le côté intérieur comprenant au moins une électrode (74) pour le ou les nerfs périphériques respectifs à moduler,
un boîtier (44) couplé mécaniquement et électriquement au bracelet, le boîtier comprenant en outre une structure d'accouplement (40, 52, 92) et un ou plusieurs contacts électriques (90), et
une ou plusieurs pistes électriques (94) s'étendant entre le boîtier et l'au moins une électrode ; et
(ii) un dispositif de neurostimulation amovible (34) présentant une surface supérieure, une surface inférieure et une paroi extérieure disposée entre celles-ci, la paroi extérieure comprenant un ou plusieurs contacts électriques (86), la paroi extérieure étant dimensionnée et formée pour être fixée mécaniquement à l'intérieur de la structure d'accouplement du boîtier ;
le ou les contacts électriques du dispositif de neurostimulation amovible étant conçus pour s'interfacer électriquement avec le ou les contacts électriques du boîtier lorsque la paroi extérieure est fixée mécaniquement à l'intérieur de la structure d'accouplement du boîtier, et
lorsque le dispositif de neurostimulation amovible est inséré dans le boîtier depuis la face intérieure du bracelet, la structure d'accouplement empêchant le dispositif de neurostimulation amovible de traverser entièrement le boîtier,
le dispositif de neurostimulation amovible étant conçu pour moduler un ou plusieurs nerfs périphériques d'un utilisateur par émission de signaux électriques.

2. Système à porter sur soi selon la revendication 1, dans lequel la structure d'accouplement est une surface de butée (52), le dispositif de neurostimulation amovible comprenant une surface de contact dont la forme et les dimensions sont adaptées à venir en contact avec la surface de butée lorsque le dispositif de neurostimulation amovible est fixé au bracelet, le bracelet étant remplaçable par un autre bracelet.

3. Système à porter sur soi selon la revendication 1, dans lequel la structure d'accouplement est une ouverture (50).

4. Système à porter sur soi selon la revendication 3, dans lequel le dispositif de neurostimulation amovible comprend en outre un écran (46), l'écran étant visible à l'intérieur de l'ouverture depuis l'extérieur du bracelet lorsque le dispositif de neurostimulation amovible est inséré dans le boîtier.

5. Système à porter sur soi selon la revendication 3, dans lequel la paroi extérieure présente la forme d'une marche, la marche comprenant une contremarche et une partie d'appui, et la circonférence de la contremarche est inférieure à la circonférence intérieure de l'ouverture.

6. Système à porter sur soi selon la revendication 5, dans lequel la partie d'appui empêche le dispositif de neurostimulation amovible de traverser entièrement le boîtier lorsque ledit dispositif est maintenu par le bracelet.

7. Système à porter sur soi selon l'une quelconque des revendications 1 à 6, dans lequel l'au moins une électrode comprend une électrode de retour ou de terre conçue pour être couplée électriquement à l'utilisateur.

8. Système à porter sur soi selon l'une quelconque des revendications 1 à 6, dans lequel l'au moins une électrode comprend au moins une première électrode et une deuxième électrode, la première électrode étant conçue pour stimuler le nerf médian de l'utilisateur et la deuxième électrode étant conçue pour stimuler le nerf radial ou ulnaire de l'utilisateur.

9. Système à porter sur soi selon l'une quelconque des revendications 1 à 6, dans lequel au moins une portion de la paroi extérieure est courbée entre les surfaces supérieure et inférieure.

10. Système à porter sur soi selon l'une quelconque des revendications 1 à 6, dans lequel au moins une portion de la paroi extérieure est plate entre les surfaces supérieure et inférieure.

11. Système à porter sur soi selon l'une quelconque des revendications 1 à 6, dans lequel le bracelet est conçu pour être serré autour d'un membre, ce qui plaque fermement l'au moins une électrode contre la peau de l'utilisateur.

12. Système à porter sur soi selon l'une quelconque des revendications 1 à 6, dans lequel les signaux électriques délivrés auxdits un ou plusieurs nerfs périphériques de l'utilisateur font varier la fréquence de rafale après une période prédéfinie.

13. Système à porter sur soi selon l'une quelconque des revendications 1 à 6, dans lequel les signaux électriques délivrés auxdits un ou plusieurs nerfs périphériques de l'utilisateur font varier la fréquence de rafale après un nombre prédéfini de rafales.

14. Système à porter sur soi selon l'une quelconque des revendications 1 à 6, dans lequel les signaux électriques délivrés auxdits un ou plusieurs nerfs périphériques de l'utilisateur font varier la fréquence d'impulsion après une période prédéfinie.

15. Système à porter sur soi selon l'une quelconque des revendications 1 à 6, dans lequel les signaux électriques délivrés auxdits un ou plusieurs nerfs périphériques de l'utilisateur font varier la fréquence d'impulsion après un nombre prédéfini de rafales.
